# EUROPEAN PATENT APPLICATION

(11) **EP 3 495 378 A1**
(43) Date of publication of application: **12.06.2019**
(21) Application number: 17206044.4
(22) Date of filing: 07.12.2017
(51) Int. Cl.: C07K 14/255, A61K 35/74, C12N 15/63, C12N 1/21

(54) **VACCINE COMPOSITIONS**

(71) Applicant: Prokarium Limited, The Science Park Keele Staffordshire ST5 5SP (GB)
(72) Inventor: Cranenburgh, Rocky, Keele Staffordshire ST5 5SP (GB); Moreira, Ricardo Neves, Keele Staffordshire ST5 5SP (GB); Salerno, Paola, Keele Staffordshire ST5 5SP (GB); Ryan, Aindrias, Keele Staffordshire ST5 5SP (GB)
(74) Representative: Gill Jennings & Every LLP

(57) **Abstract**

A genetically engineered non-natural bacterium comprising one or more of the following genetic modifications:
(i). one or more heterologous genes encoding factors that prevent cell division, the expression of which are under the control of an inducible promoter activated when the bacterium is internalised into the intracellular vesicle of an animal cell;
(ii). one or more endogenous factors that promote cell division, the expression of which are genetically modified to be repressed and/or prevented when the bacterium is internalised into the intracellular vesicle of an animal cell; and/or
one or more endogenous factors that repress cell division, the expression of which are genetically modified to be under the control of an inducible promoter activated when the bacterium is internalised into the intracellular vesicle of an animal cell.

SEQUENCE LISTING

<110>  Prokarium Limited

<120>  Vaccine Compositions

<130>  JWJ02382GB

<160>  39

<170>  PatentIn version 3.5

<210>  1
<211>  708
<212>  DNA
<213>  Salmonella typhimurium

<400>  1
atgtcaaaca cgccaatcga gcttaaaggc agtagcttca ccttatcagt ggttcatttg      60

catgaagcgg aaccagaggt tattcgtcag gcgttagaag acaaaatagc gcaggctcct     120

gcgtttttaa aacacgcccc tgtcgttatt aacgttagcg gtcttgaaag cccggtaaac     180

tggccggaac tgcataagat cgtgacctca accggcttac gcattatcgg cgtgagcggc     240

tgtaaagacg ccagcctgaa agtagaaatc gatcggatgg gtcttccttt actgactgaa     300

ggtaaagaga aagcggttcg gcctgcgccc gttgagccgg caacgcccag cgaaccgccg     360

caaaacgcta atcccatcac aaaaacgcga ttaattgatg ttccggttcg ttccggtcag     420

cgcatttatg ctccacaatg tgatctgatt gttacaagtc acgtcagcgc tggcgcagag     480

cttatcgctg acggcaatat ccatgtctat ggcatgatga gaggtcgcgc gctggcagga     540

gcgagcggcg atcgggaagc gcaaattttt tgtacccatc tgacggcaga actggtctct     600

atcgcaggtg tttactggct gagtgataaa atcccggcag aattttatgg caaagcggcg     660

cgcctgcgtt tagcagacaa cgctttgaca gttcaaccgt tgaattaa                  708

<210>  2
<211>  222
<212>  DNA
<213>  Salmonella typhi

<400>  2
atgaaaacat tacctgtatt acccgggcag gcggccagtt ctcgcccgtc tcctgttgaa      60

atctggcaga tactgctgtc ccgactgctg gaccagcact atggcctcac actgaatgac     120

acaccttttg ccgatgaacg tgtgattgag cagcatattg aggcaggcat ttcactgtgt     180

gatgcggtga actttctcgt ggaaaaatac gcgctggtgt aa                        222

<210>  3
<211>  510
<212>  DNA
<213>  Salmonella typhimurium

<400> 3
atgtacactt caggttatgc aaatcgttct tcgtcatttc ctaccactac ccacaacgct 60

gcgcgcaccg ctacggaaaa tgccgcggca ggactggtca gtgaagttgt ctaccacgaa 120

gaccagccca tgatggcgca actcctgctt ttgcctttac tccgtcagtt aggccaacaa 180

tcacgctggc agctctggct cacgccgcag caaaagctca gccgtgaatg ggtacagtct 240

tcaggtttgc cattaacgaa agtgatgcaa attagccagc ttgcgcctcg tcatacgctg 300

gagtcgatga tccgcgcttt gcgtacagga aattacagcg tggtaattgg ttggatgact 360

gaagaactga cagaagaaga acatgccagc ctggttgaag cagcgaaggt aggtaatgcg 420

gtagggttta tcatgcgccc tgtacgtgcg cacgctttac ccaggagaca gcattccggg 480

ctaaaaattc actctaattt gtatcattaa 510

<210> 4
<211> 597
<212> DNA
<213> Salmonella typhi

<400> 4
atggcagaaa aacaaactgc gaaaaggaat cgtcgcgaag aaatacttca gtcactggcg 60

ctgatgcttg aatccagcga tggaagccaa cgcatcacga cggcaaaact ggcggcctcg 120

gtcggcgttt ccgaagcggc gctataccgc catttcccca gcaagacccg catgttcgat 180

agcctgattg agtttatcga agatagtctg attacccgta tcaatctgat tttaaaagat 240

gaaaaagaca ccagcacgcg tttgcgcctg attgtgttac tgattctggg ttttggcgaa 300

cgcaaccctg gcctgacgcg cattcttacc gggcatgcgc tgatgtttga acaggatcgc 360

ctgcaggggc gcattaatca gctttttgaa cgcatcgaag cgcagctccg ccaggtgcta 420

cgagaaaaaa gaatgcgtga aggcgaaggc tacacgacgg ataaaaatct gctggcaagc 480

cagctgttag ccttctgtga aggtatgctt tcacggttcg tgcgtagcga atttaaatac 540

cgcccaacgg acgatttcga cgcccgctgg ccgctcattg ccgcgcagtt gcagtaa 597

<210> 5
<211> 1125
<212> DNA
<213> Salmonella typhi

<400> 5
atgcaaagtc tttcccctac atcgcgttat ctccaggcac tcaacgaagg cacccatcag 60

cctgacgacg tacagaaaga ggcggtcgac cgtctggaaa ccctttatca ggcgctcacc 120

gcgaaaaaat cgtcggctac gccacccggc ggtctgatag cgcgactggg gaaattatta 180

ggcaaaaacg aaccggacgc gcaaataccg gttcgcgggc tgtatatgtg ggggggcgtg 240

ggtcgcggca aaacctggct catggatctc ttctaccaca gcctgccggg tgagcgaaaa 300

ttgcgcctgc attttcatcg gtttatgctg cgcgtgcatg aagagcttac cgcgctacag 360

ggacagatcg atccgttaga cattattgcc gatcggttta aaaccgagac ggacgtactg 420

tgtttcgatg agtttttcgt caccgatatt accgacgcca tgctgctggg cggtttaatg 480

aaagcgctgt ttgcacgcgg cattacgctg gtcgcgacct ccaatattcc gccggaggaa 540

ctctaccgca acggtttgca acgcgcccgc ttcctgccgg ctatcgatgc cattaaacag 600

cactgcgaca ttatgaatgt ggacgccggc gtcgattacc ggctgcggac gctgacgcag 660

gcccatttat ggctaacgcc gcttaatgat gaaacccggc ggcaaatgga taaactgtgg 720

ctggcgttag cgggggccgc acgagaacat gctccgacgc tggaaatcaa tcatcgccct 780

ttatccaccc tgggcgttga aaaccaaacg ttagcggcct ctttcgctac gctgtgcgtc 840

gaggctcgca gccagcatga ttacattgcg ctctcaaggt tatttcacac ggttctgttg 900

tttgacgtgc cggtcatgac gccgctgatg gagaacgaag cgcgccgctt tattgcgctg 960

gtggatgagt tttacgagcg ccacgttaag ctggtggtca gcgccgccgc gccgttatat 1020

gagatttatc agggggagcg actcaagttt gaatttcagc gctgtctgtc gcgcctgcaa 1080

gagatgcaga gtgcggagta tctcaagcgg gagcatatgc cgtaa 1125

<210> 6
<211> 672
<212> DNA
<213> Salmonella typhi

<400> 6
atgcagtacc cgattaacga gatgttccag accctacaag gcgagggtta ttttaccggc 60

gtccccgcta tttttattcg tttacaggga tgtccggtcg gctgtgcctg gtgtgatact 120

aagcatacct gggataaact gagcgatcgg gaagtgtctc tgttcagcat tctggcaaaa 180

accaaagaga gcgacaaatg gggtgctgcg agcagtgaag acttgctggc agtaattaat 240

cgtcagggct ataccgcgcg tcacgttgtc atcaccggtg gcgaaccctg tattcacgac 300

ctcatgccgc tcaccgatct gcttgaaaag agtggtttta gttgccagat tgaaaccagc 360

gggacgcacg aagtgcgctg tacgcccaat acctgggtca cggtttcacc aaaagtgaat 420

atgcgcgggg gatatgacgt gctttcccag gcgctggagc gcgccaatga aatcaagcat 480

cccgttgggc gagttcgtga tattgaggcg ctggatgaac tgttagccac gttgagcgat 540

gataaaccgc gcgtgattgc tttgcagccc attagccaga aagaagacgc gacgcgtctg 600

tgtattgaaa cgtgtattgc gcgtaactgg cggctgtcta tgcaaacgca taaatattta 660

aatatcgcct aa 672

<210> 7
<211> 53
<212> DNA

<213>  Escherichia coli

<400>  7
tttctggtga cgtttggcgg tatcagtttt actccgtgac tgctctgccg ccc          53


<210>  8
<211>  1152
<212>  DNA
<213>  Salmonella typhi

<400>  8
atgtttgaac ctatggaact aaccaacgac gcggtgatta cagtcatcgg cgtcggtggt          60

ggcggcggta acgctgttga acacatggtg cgcgagcgca tcgaaggtgt tgaattcttc         120

gcggtgaata ccgccgcgca ggcgttgcgt aagacggcgg ttggccagac cattcaaatt         180

ggtagcggca ttaccaaagg tctgggcgcc ggcgctaacc cggaagtcgg ccgtaatgcg         240

gcggacgaag accgtgaagc gctgcgtgcg gcgcttgaag gcgcagacat ggtatttatc         300

gcggcaggca tgggcggcgg taccggaacc ggcgcggcgc cagtggttgc tgaagtggca         360

aaagatttag gtattctgac cgttgccgtg gtgactaagc ctttcaattt tgaaggcaaa         420

aagcgcatgg catttgcgga gcaaggtatc accgagctgt ccaagcatgt ggactcgttg         480

atcaccatcc cgaacgacaa gctgctgaaa gtgctgggcc gtggcatctc cctgctcgac         540

gcgttcggcg cggctaacga cgtgctgaaa ggcgcggttc agggtatcgc agagctgatc         600

acccgcccgg gcctgatgaa cgtagacttt gctgacgtgc gtaccgtgat gtctgaaatg         660

ggctacgcaa tgatgggctc cggcgtggcg agcggtgaag accgtgcgga agaagcggct         720

gaaatggcta tttcttctcc gctgctggaa gatatcgacc tgtccggcgc gcgcggcgtg         780

ttggtcaaca ttacggcggg cttcgacctg cgtctggatg agttcgaaac cgtcggtaac         840

accatccgtg cctttgcgtc ggataacgcc accgtggtta tcggtacctc tctggacccg         900

gatatgaacg acgaactgcg cgtgaccgtt gttgccactg gaattggtat ggacaagcgt         960

cctgaaatca ctctggtcac caataaacag gtacagcagc cggtgttgga tcgttatcag        1020

cagcacggta tggcgccgct gacgcaggag caaaaaacgg tcgcgaaagt ggttaacgac        1080

aatgcgccac aggcggcgaa agagccggat tatctggata tccccgcatt cctgcgtaag        1140

caagctgatt aa                                                            1152


<210>  9
<211>  1152
<212>  DNA
<213>  Salmonella typhi

<400>  9
atgtttgaac ctatggaact aaccaacgac gcggtgatta cagtcatcgg cgtcggtggt          60

ggcggcggta acgctgttga acacatggtg cgcgagcgca tcgaaggtgt tgaattcttc         120

gcggtgaata ccgacgcgca ggcgttgcgt aagacggcgg ttggccagac cattcaaatt 180

ggtagcggca ttaccaaagg tctgggcgcc ggcgctaacc cggaagtcgg ccgtaatgcg 240

gcggacgaag accgtgaagc gctgcgtgcg gcgcttgaag gcgcagacat ggtatttatc 300

gcggcaggca tgggcggcgg taccggaacc ggcgcggcgc cagtggttgc tgaagtggca 360

aaagatttag gtattctgac cgttgccgtg gtgactaagc ctttcaattt tgaaggcaaa 420

aagcgcatgg catttgcgga gcaaggtatc accgagctgt ccaagcatgt ggactcgttg 480

atcaccatcc cgaacgacaa gctgctgaaa gtgctgggcc gtggcatctc cctgctcgac 540

gcgttcggcg cggctaacga cgtgctgaaa ggcgcggttc agggtatcgc agagctgatc 600

acccgcccgg gcctgatgaa cgtagacttt gctgacgtgc gtaccgtgat gtctgaaatg 660

ggctacgcaa tgatgggctc cggcgtggcg agcggtgaag accgtgcgga agaagcggct 720

gaaatggcta tttcttctcc gctgctgaaa gatatcaagc tgtccggcgc gcgcggcgtg 780

ttggtcaaca ttacggcggg cttcgacctg cgtctggatg agttcgaaac cgtcggtaac 840

accatccgtg cctttgcgtc ggataacgcc accgtggtta tcggtacctc tctggacccg 900

gatatgaacg acgaactgcg cgtgaccgtt gttgccactg gaattggtat ggacaagcgt 960

cctgaaatca ctctggtcac caataaacag gtacagcagc cggtgttgga tcgttatcag 1020

cagcacggta tggcgccgct gacgcaggag caaaaaacgg tcgcgaaagt ggttaacgac 1080

aatgcgccac aggcggcgaa agagccggat tatctggata tccccgcatt cctgcgtaag 1140

caagctgatt aa 1152

<210> 10
<211> 945
<212> DNA
<213> Salmonella typhi

<400> 10
atgtttgaac ctatggaact aaccaacgac gcggtgatta cagtcatcgg cgtcggtggt 60

ggcggcggta acgctgttga acacatggtg cgcgagcgca tcgaaggtgt tgaattcttc 120

gcggtgaata ccgacgcgca ggcgttgcgt aagacggcgg ttggccagac cattcaaatt 180

ggtagcggca ttaccaaagg tctgggcgcc ggcgctaacc cggaagtcgg ccgtaatgcg 240

gcggacgaag accgtgaagc gctgcgtgcg gcgcttgaag gcgcagacat ggtatttatc 300

gcggcaggca tgggcggcgg taccggaacc ggcgcggcgc cagtggttgc tgaagtggca 360

aaagatttag gtattctgac cgttgccgtg gtgactaagc ctttcaattt tgaaggcaaa 420

aagcgcatgg catttgcgga gcaaggtatc accgagctgt ccaagcatgt ggactcgttg 480

atcaccatcc cgaacgacaa gctgctgaaa gtgctgggcc gtggcatctc cctgctcgac 540

gcgttcggcg cggctaacga cgtgctgaaa ggcgcggttc agggtatcgc agagctgatc 600

acccgcccgg gcctgatgaa cgtcgacttt gctgacgtgc gtaccgtgat gtctgaaatg 660

ggctacgcaa tgatgggctc cggcgtggcg agcggtgaag accgtgcgga agaagcggct 720

gaaatggcta tttcttctcc gctgctggaa gatatcgacc tgtccggcgc gcgcggcgtg 780

ttggtcaaca ttacggcggg cttcgacctg cgtctggatg agttcgaaac cgtcggtaac 840

accatccgtg cctttgcgtc ggataacgcc accgtggtta tcggtacctc tctggacccg 900

gatatgaacg acgaactgcg cgtgaccgtt gttgccactg gataa 945

<210> 11
<211> 1272
<212> DNA
<213> Salmonella typhi

<400> 11
atgacagata aacgcaaaga tggctcgggc aaattgttgt actgctcttt ttgcggcaaa 60

agccagcatg aagtgcgcaa gctgattgcc ggtccatccg tgtatatctg cgacgaatgc 120

gtcgatttat gtaacgacat tattcgcgaa gaaattaaag aagttgctcc gcaccgtgaa 180

cgtagtgcgc tgccgacgcc gcatgaaatt cgtactcacc tggacgatta cgttatcggc 240

caggagcagg cgaaaaaagt gctggcggtg gcggtctata accactacaa gcgtctgcgt 300

aacggcgata ccagcaatgg cgtcgagtta ggcaaaagca acattctgct gactggaccg 360

accggttccg gtaaaacgct gctggcggaa acgctggcgc gcttgctgga tgtgccgttc 420

actatggcgg atgcgaccac gctgaccgaa gcgggttacg tgggggaaga cgtcgagaat 480

atcattcaga aactgttgca gaaatgcgac tacgacgtgc aaaaagcgca gcgtgggatt 540

gtctacattg atgaaatcga taagatttcg cgtaaatcag acaatccgtc cattacccgc 600

gatgtttccg gcgaaggcgt acagcaggcg ttgctgaaac tgatcgaagg caccgtcgcc 660

gcggttccac cgcagggcgg tcgcaaacat ccgcagcagg agttcttaca ggtagatacc 720

tctaagattc tgtttatctg cggcggcgcg tttgccggtc tggataaagt gatcgctaac 780

cgtgttgaaa ccggctccgg cattggtttt ggcgcgacgg tgaaagcgaa gtccgacaaa 840

gccagtgaag gcgagctgtt gtcgcaggtt gaaccggaag atttgatcaa atttggtctg 900

attcctgagt ttatcggtcg tctgccagtg gtggcgacgc tgaacgaact cagcgaagaa 960

gcgctggttc aaatcctgaa agagccgaaa aatgcgctga ccaagcagta tcaggcgctg 1020

tttaacctgg aaggcgtcga tctggaattc cgtgacgaag cgctgaacgc tatcgccagg 1080

aaagcgatgg cgcgtaaaac tggtgcccgt ggcctgcgtt ctatcgtcga agcggcgctg 1140

ctggatacca tgtacgattt gccatctatg gaagacgtcg aaaaagtggt gattgacgag 1200

tccgttattg ccggtcagag taagccgttg ctgatttacg gcaaaccgga agcgcaggct 1260

tctggcgaat aa 1272

<210> 12
<211> 624
<212> DNA
<213> Salmonella typhi

<400> 12
atgtcataca gcggagaacg agataatttg gcccctcata tggcgctggt gccgatggtc 60

attgaacaga cctcacgcgg tgagcgctct tttgatatct attctcgtct acttaaggaa 120

cgcgtcatat ttctgaccgg ccaggtcgaa gaccatatgg ctaacctgat cgtggcgcag 180

atgctgttcc tggaagcgga aaacccggaa aaagatatct atctgtacat taattctcct 240

ggcggcgtaa ttactgcggg gatgtccatc tatgacacca tgcagtttat taagccagac 300

gtcagcacca tttgtatggg acaggcggcc tctatggggg cgtttctgct gactgccggg 360

gcgaaaggca aacgtttctg cttgccgaac tctcgcgtca tgatccacca gccgctgggc 420

ggctatcagg gccaggcgac ggatattgaa attcacgccc gcgaaatttt gaaagtaaaa 480

gggcgcatga atgaacttat ggcgcatcat acgggtcaat ctcttgagca gattgaacgt 540

gatactgagc gcgatcgctt cctctccgcg cctgaagcgg tagagtacgg tttggttgac 600

tcaattttga cccatcgtaa ttaa 624

<210> 13
<211> 813
<212> DNA
<213> Salmonella typhi

<400> 13
   atggcacgca ttattgttgt tacttcgggt aaagggggcg ttggcaagac cacctccagc 60
   gcggccatcg ctacaggttt ggcccagaag ggaaagaaaa ctgtcgtcat tgattttgat 120
   atcggactgc gtaacctcga tctgattatg gggtgcgaac gtcgtgtcgt ttacgatttt 180
   gtaaacgtca ttcagggcga tgcgacactg aatcaggcgc tgatcaaaga taagcgtact 240
   gaaaatctct tcattcttcc ggcgtcgcag acccgggata aagacgcgct aacgcgcgaa 300
   ggcgtcgcta aggtactgga ctcactgaaa gcaatggact ttgagttcat tgtttgcgac 360
   tcgccggcgg gtatcgaaac cggggcgctg atggcgctct attttgccga tgaagcgatc 420
   atcacgacca acccggaagt ctcttctgtc cgtgactcgg accgtattct gggtattctg 480
   gcatcgaaat ctcgtcgcgc agaaaatggc gaagaaccga ttaaagaata tctcctgttg 540
   acgcgctaca atccaggccg cgtcaataaa ggcgacatgc tcagcatgga agatgtactg 600
   gagattctgc gtattaaact cgtcggtgtg atcccggaag atcaatccgt actgcgcgca 660
   tcgaaccagg gcgaaccggt gattcttgac gccactgcgg atgcgggtaa agcctatgca 720
   gataccgtag atcgtctgtt gggagaagaa cgtcctttcc gcttcattga agaagagaag 780
   aaaggtttcc tcaaacgcct gttcggagga taa 813

<210>  14
<211>  267
<212>  DNA
<213>  Salmonella typhi

<400>  14
atggcattac tggatttttt tctctcgcgg aaaaagagta ccgctaatat tgcaaaagag      60

cggttgcaaa tcattgtcgc ggaacgtcgc cgtagcgatg ccgaaccgca ttatttaccg     120

cagttacgta aagatattct tgaggtcatt tgtaaatacg tacaaatcga tccggaaatg     180

gttaccgtcc agcttgagca aaaagatggt gatatctcca tccttgaact taacgtaacg     240

ctgccggaag ccgaagagtc aaaataa                                         267


<210>  15
<211>  1152
<212>  DNA
<213>  Salmonella typhi

<400>  15
atgtttgaac ctatggaact aaccaacgac gcggtgatta cagtcatcgg cgtcggtggt      60

ggcggcggta acgctgttga acacatggtg cgcgagcgca tcgaaggtgt tgaattcttc     120

gcggtgaata ccgacgcgca ggcgttgcgt aagacggcgg ttggccagac cattcaaatt     180

ggtagcggca ttaccaaagg tctgggcgcc ggcgctaacc cggaagtcgg ccgtaatgcg     240

gcggacgaag accgtgaagc gctgcgtgcg gcgcttgaag gcgcagacat ggtatttatc     300

gcggcaggca tgggcggcgg taccggaacc ggcgcggcgc cagtggttgc tgaagtggca     360

aaagatttag gtattctgac cgttgccgtg gtgactaagc ctttcaattt tgaaggcaaa     420

aagcgcatgg catttgcgga gcaaggtatc accgagctgt ccaagcatgt ggactcgttg     480

atcaccatcc cgaacgacaa gctgctgaaa gtgctgggcc gtggcatctc cctgctcgac     540

gcgttcggcg cggctaacga cgtgctgaaa ggcgcggttc agggtatcgc agagctgatc     600

acccgcccgg gcctgatgaa cgtcgacttt gctgacgtgc gtaccgtgat gtctgaaatg     660

ggctacgcaa tgatgggctc cggcgtggcg agcggtgaag accgtgcgga agaagcggct     720

gaaatggcta tttcttctcc gctgctggaa gatatcgacc tgtccggcgc gcgcggcgtg     780

ttggtcaaca ttacggcggg cttcgacctg cgtctggatg agttcgaaac cgtcggtaac     840

accatccgtg cctttgcgtc ggataacgcc accgtggtta tcggtacctc tctggacccg     900

gatatgaacg acgaactgcg cgtgaccgtt gttgccactg gaattggtat ggacaagcgt     960

cctgaaatca ctctggtcac caataaacag gtgcagcagc cggtgttgga tcgttatcag    1020

cagcacggta tggcgccgct gacgcaggag caaaaaacgg tcgcgaaagt ggttaacgac    1080

aatgcgccac aggcggcgaa agagccggat tatctggata tccccgcatt cctgcgtaag    1140

caagctgatt aa                                                          1152

<210>  16
<211>  1263
<212>  DNA
<213>  Salmonella typhi

<400>  16
atgatcaagg cgacggacag aaaactggta gtaggactgg agattggcac cgcgaaggtt      60

gccgctttag taggggaagt tctgcccgac ggtatggtca atatcattgg cgtgggcagt     120

tgcccatcgc ggggtatgga taaaggcggg gtgaatgacc ttgagtcagt ggtgaaatgc     180

gttcagcgcg ccattgacca ggcggaattg atggctgatt gccagatctc ctcggtatat     240

ctggcgcttt ccggcaaaca tattagctgt cagaatgaaa ttggtatggt gcccatttcc     300

gaagaggaag tgacgcagga agatgtggaa aacgtcgtgc atactgcgaa gtcagtgcgc     360

gtgcgcgatg aacatcgcgt gctgcatgtg attcctcagg agtacgccat tgactaccag     420

gaaggcatta aaaatccggt cggtctttcc ggcgtgcgta tgcaggcgaa agtacatttg     480

attacctgcc acaacgatat ggcaaaaaac attgtcaaag ctgttgaacg ttgtggtctg     540

aaagttgacc aactgatatt tgccggactg gcggccagtt attccgtgtt aacggaagat     600

gaacgtgaac tgggcgtctg tgtcgtggat atcggcggtg gtacaatgga tatcgccgtt     660

tatactggcg gcgcgctgcg tcataccaaa gtgatccctt acgcggggaa cgtggtgacc     720

agcgatatcg cttatgcctt tggtacgccg ccgagcgatg ctgaagccat taaagttcgc     780

cacggttgcg cgctgggttc cattgtcggt aaagacgaaa gcgttgaagt accgagcgta     840

ggcgggcgtc cgccgcgcag cctgcaacgt cagacgctgg cggaagtgat tgagccgcgt     900

tacaccgagc tgctcaatct ggtcaacgaa gagatattgc aattacagga acagcttcgc     960

caacaagggg taaaacatca cctggcggcg ggcattgtgt taaccggcgg cgcggcgcaa    1020

atcgaagggc ttgctgcttg cgctcagcgc gtgtttcata cgcaggtgcg tatcggcgcg    1080

ccgctgaata tcaccggtct gacggattat gcccaggagc cgtattactc aacggcggtg    1140

ggattgcttc actacgggaa agagtcccac ttaaacggtg aagcagaagt ggaaaaacgt    1200

gttacggcat cagttggctc gtggatcaag cgacttaata gctggctgcg aaaagagttt    1260

taa                                                                   1263

<210>  17
<211>  987
<212>  DNA
<213>  Salmonella typhi

<400>  17
atgatgcagg atttgcgtct gatattaatc attgttggcg cgatcgccat aatcgcttta      60

ctggtacatg gtttctggac cagccgtaaa gaacgatctt ctatgttccg cgatcgtcca     120

ctaaaacgca tgaaatcgaa acgcgacgac gactcgtatg acgacgatgt cgaagaagac 180

gaaggcgtgg gtgaagttcg tgttcaccgc gtaaatcatg cccccggcca gtcgcaggag 240

catgatgccc ctcgtcagtc gccgcaacac cagtaccagc cgccttatgc gtctgcgcaa 300

ccgcgtccag cggcgccgcc gcagccgcaa gcgccgatgc aacagccggt tcagcaaccg 360

gtccaacctg cgccgcagcc tcagcaggtt cagccatctg cgccgccggt acagccgccg 420

caacagcagc ctgcaccgcc gtcgcaagcg ccgcagcctg ttgcgcagcc cgcgccgccg 480

ccatcagcgc aaaccttcca gcctgcggaa cctgtggttg aggcggaacc ggtagtggaa 540

gaagcgccgg ttgtggaaaa accgcagcgt aaagaggcgg tcatcattat gaacgtcgcg 600

gcccatcatg gcagcgagct taatggtgaa gtgttactga acagtattca gcagtcaggc 660

tttaaatttg gcgatatgaa tattttccat cgtcatttga gcccggacgg cagcggcccg 720

gccttgttca gtctcgcgaa tatggtcaat ccgggaacgt tcgatcctga aatgacggac 780

ttcactacgc ctggcgtcac tatctttatg caggtaccgt cttacggcga tgcgctgcag 840

aatttcaagc tgatgctgca atccgcgcag catatcgctg atgaggtcgg tggcgtcgtg 900

ctggatgacc agcgtcggat gatgacgccg cagaaactgc gcgaatatca ggaccgcatc 960

cgcgaggtga tggacgctaa cgcctaa 987

<210> 18
<211> 831
<212> DNA
<213> Salmonella typhi

<400> 18
atgtcgcagg ctgcgctgaa cacgcgaaac agtgatgaag agacgccttc ttcacgccgc 60

aataatggaa cgcgtcttgc aggaatatta ttcctgctga cggtactgtg caccgtgttt 120

gttagcgggt gggtcgtgtt agggtggatg gaagatgcgc agcgtttacc gttatcaaag 180

ctggtattaa ccggcgagcg tcactacacg cgtaacgatg atatccgtca ggcgatactg 240

gctctgggcg cgccgggaac cttcatgacg caggatgtga acattatcca gagccagatt 300

gagcgtttgc cgtggattaa acaggccagc gtcagaaaac agtggcctga tgaattgaag 360

attcatctgg ttgaatatgt gccgattgcg cgttggaatg atcaacatat ggtcgatgct 420

gaagggaaca ccttcagcgt gccgtcagac cgcatcggca aacaggtttt acccatgttg 480

tacggcccgg aaggcagcgc gagtgaagtg ttgcagggat accgcgagat ggggcaggtg 540

ctggctaagg ataaattcac tctaaaggaa gcggcgatga ccgcacgtcg ctcctggcaa 600

ttgacgctta ataacggcat taagctcaat ctcggcaggg gcgatacgat gaaaaggttg 660

gctcgctttg tagaactcta tccggtttta cagcagcagg cgcaaaccga tggcaaacgg 720

attagctacg ttgatttgcg ttatgactca ggtgcggcag ttggctgggc gccgctcccc 780

cctgaggaat ctaatcagca acagaatcag gcacaggcag aacaacaata a 831

<210> 19
<211> 366
<212> DNA
<213> Salmonella typhi

<400> 19
atgatcagca gagtgacaga agccctaagc aaagttaagg gatcgatagg aagcaacgag 60

cgccatgcct tgcctggcgt gatcggtgac gatcttttgc ggttcgggaa gctgccactc 120

tgcttgttca tttgcatcat tttaacggcg gtgacggtgg tcacgacggc gcaccatact 180

cgtttactca ccgctcagcg tgaacaactg gttctggagc gcgatgcatt ggacattgaa 240

tggcgcaacc tgatccttga agaaaatgcg ctcggcgatc acagccgggt agagcggatc 300

gcaacggaaa agctgcaaat gcagcatgtt gatccgtccc aagaaaatat cgtagtgcaa 360

aaataa 366

<210> 20
<211> 1767
<212> DNA
<213> Salmonella typhi

<400> 20
atgaaagcag cggcaaaaac gcaaaaatcg aagcgccagg aagaacagac caacttcatc 60

agttggcgtt ttgcgttgct gtgcggctgt attttactgg cgctggtttt tttacttgga 120

cgcgcggcgt ggctacagat tatcgccccg gatatgctgg tgcgtcaggg cgatatgcgt 180

tcgctgcgtg ttcaggaagt ctccacctca cgcggtatga ttaccgaccg ttccgggcgt 240

ccactggcgg tcagcgtacc ggttaaggcg atctgggccg acccgaaaga agttcatgac 300

gccggcggca tcagcgtcgg cgatcgctgg aaagcgcttt ctactgcgct caatattccg 360

ctcgatcagc tctccgcccg tatcaacgcc aacccgaaag ggcgttttat ttatctggcg 420

cgccaggtaa atcctgacat ggcggactac atcaaaaaac tcaaactgcc ggggattcat 480

ttgcgtgaag aatcacgccg ctactacccg tcaggagaag taaccgctca cctcatcggt 540

tttaccaacg ttgacagtca ggggattgaa ggcgttgaaa agagctttga taagtggctt 600

accggtcagc ctggcgagcg aatcgtgcgt aaagaccgct atggccgcgt gattgaggat 660

atctcctcca ctgacagtca ggcagcgcac aacctggcgt taagcattga tgaacgctta 720

caggcgctgg tttaccgcga actgaacaac gcggtggcgt ttaacaaggc ggaatccggt 780

agcgcggtgc tggtcgatgt caataccggt gaagtactgg cgatggcgaa tagcccttcc 840

tataacccca acaaccttgc cggtacgccg aaagacgcga tgcgtaaccg taccatcacc 900

gacgtgtttg aaccgggttc tacggttaag ccgatggtgg tgatgactgc gcttcagcgc 960

ggtatcgtca acgaaaatac ggtgttgaat accgttccat accgaattaa cggccacgaa 1020

attaaagacg tggcgcgcta cagcgaatta accctgactg gggttctaca gaagtcgagt 1080

aacgtcggtg tatccaaact ggcgttagcg atgccgtcct cagcgttagt agatacttac 1140

tcacgttttg ggctaggaaa ggcgaccaat ttggggttgg tcggagaacg cagtggctta 1200

tatcctcaaa aacaacggtg gtccgacata gagagggcca ccttctcttt cggctacggg 1260

ctaatggtaa cgccgttaca gttagcgcga gtttatgcaa cgatcggcag ctatggcatt 1320

tatcgcccgc tgtcgattac caaagttgat ccgcctgtac ccggcgaacg catcttccct 1380

gaatccaccg tacgcaccgt ggtgcatatg atggaaagcg tggcgctgcc gggcggcggc 1440

ggcgtgaagg cggcaattaa aggctaccgt atcgcgatta aaaccggtac ggcgaaaaaa 1500

gtggggccgg acggtcgcta catcaacaaa tacattgctt acaccgcagg cgtcgcgcct 1560

gcgagtcagc cgcgcttcgc gctggtggtt gtcatcaacg atccgcaggc gggtaaatac 1620

tacggcggcg ccgtttccgc gccggtattt ggtgccatca tgggcggcgt tctgcgcacc 1680

atgaacatcg aaccggacgc gctggcaacg ggcgaaaaaa atgaattcgt aattaatcaa 1740

ggcgagggaa caggtggcag atcgtaa 1767

<210> 21
<211> 996
<212> DNA
<213> Salmonella typhi

<400> 21
gtggcacaac gagattatgt acgtcgcggc caaccggctc cttcgcggcg caaaaagagc 60

acctcacgga actcacggaa gaagcaaagc aacatgcctg ccgtctcgcc ggcaatggtc 120

gcgattgcgg cagccgtcct tgtgaccttt atcggcggtc tgtacttcat tactcaccat 180

aagaaagaag agtccgaaac gctacaaaac cagaaagtca ccggcaatgg gcttccgcca 240

aaaccggaag agcgctggcg ttacatcaaa gagctggaaa gtcgccagcc gggcgtgcgt 300

acgccaacgg aaccgtccgc cggcggcgaa gtcatgaatc cgaatcagct caccagcgag 360

cagcgtcaac tcctggagca gatgcaggcg gatatgcgtc agcagccgac acaacttaac 420

gaagtgccgt ggaacgagca gacgccagaa caacgccagc aaacgctaca gcgccagcgt 480

caggcgcagc agcagcagca gcagcagcag cagcagcagt gggctcaaac gcagcctgtt 540

cagcaaccac gcacgcagcc gcgggtaaac gaacagccgc aaacgcgcac ggtacagtcc 600

gccccggcgc aacccgcgcg ccaaagtcag ccgccgaagc agacggcctc gcagcaacct 660

tatcaggatt tgctgcaaac gccggcgcat accagcgcgg cagcgccaaa agccgcgccc 720

attacgcgcg cgcctgaagc gccgaaggcg acagcagaga aaaaagatga acgtcgctgg 780

atggtgcaat gtggttcatt caaaggcgcc gagcaggcag aaagcgtccg cgctcagctg 840

gcgtttgaag gctttgactc caaaattacc accaataacg gctggaatcg cgtggttatc 900

gggccggtga aaggtaaaga aaatgccgac agcaccatta accgtctgaa gatggcgggc 960

catacaaact gcattcgact cgccaccggg ggttaa 996

<210> 22
<211> 28
<212> DNA
<213> Salmonella enterica

<400> 22
ggtgcgcata atgtatatta tgttaaat 28

<210> 23
<211> 903
<212> DNA
<213> Salmonella typhi

<400> 23
atgacggagg tcgcgctttc tctggatgtc tcgcggttcc tgcgctattt aggcgtggag 60

cgccagctta gccccattac cctgcagaac taccagcgtc agcttgatgc cattatcgct 120

ttagccggag aaaccgggct gaaaagctgg caacaatgtg acgctgcgat tgtgcgcagt 180

tttgcggtgc gcagtcggcg taaagggctt ggccctgcca gtctggcgct acgtctctcg 240

gcattgcgaa gcttttttga ctggttagtc agtcagggcg aattgaaagc taacccggcg 300

aagggcgtct ccgcaccgaa agcgccgcgt catctgccga aaaatattga tgtcgacgac 360

gttaaccgcc tgctggacat cgatcttaac gatccgctcg ccgtacgtga ccgggcgatg 420

ctggaggtca tgtacggcgc ggggctgcgt ttatccgaac tggttggcct ggatataaag 480

cacctcgatc tcgataccgg agaagtgtgg gtaatgggta aaggcagcaa agagcgacgc 540

ctgccgattg gccgtaacgc ggtgacgtgg attgagcact ggctggatct gcgcggcctg 600

ttcgccagcg atgaagaggc gttattcctg tcgaagctcg gcaagcgtat ctccgcgcgc 660

aatgtccaaa aacggttcgc cgaatggggc ataaagcagg ggctaaacag ccacgtgcat 720

ccacataagc tgcgccattc gttcgcgacc catatgctgg aatcgagcgg cgatctgcgc 780

ggcgtgcaag agctgctggg tcatgccaac ctttccacca cccaaattta tactcatctt 840

gattttcaac atcttgcttc agtgtacgac gcggcgcatc cgcgcgctaa acgggggaaa 900

taa 903

<210> 24
<211> 897
<212> DNA
<213> Salmonella typhi

<400> 24
atggaacagg atttagcgcg tattgagcag tttctggatg ccctgtggct ggaacgaaat 60

ctggcggaaa atacattaag cgcgtatcgt cgcgatttat cgatggtcgt tgcgtggttg 120

catcatcgcg gaaaaacgct tgcgacggcg caggctgatg atttacaaac gctattggct 180

gaacgggtgg agggcggata caaagcgacc agctccgcac gcctgttaag cgctatgcgg 240

cgctttttcc agcatctgta ccgtgaaaag tatcgtgagg atgatccgag cgcacagttg 300

gcctcgccta agcttccgca gcgtttaccg aaagacctta gcgaggcgca ggttgaacgg 360

ctcttgcagg cgccgcttat tgatcaacct ttggagttgc gcgataaagc tatgttggaa 420

gtgctgtatg ccaccggttt gcgcgtgtcg gaactggtgg ggctgacaat gagcgatatc 480

agtctgcggc agggcgtggt gcgggttatc ggtaaaggca ataaagagcg gttggtgccg 540

ttaggcgaag aggcggtata ttggctggaa acgtatctgg agcatggcgg tccctggtta 600

ctcaatggcg tatcgattga cgtattattc cccagtcagc gtgcgcagca gatgacgcga 660

cagaccttct ggcaccgtat taaacattat gcggtactgg ctggaattga tagcgaaaaa 720

ctatcgccgc atgttttacg tcacgctttc gccacgcatt tgctgaatca cggcgccgac 780

ttgcgcgttg tacaaatgct acttgggcat agcgatcttt cgacgacaca aatttatacg 840

catgtcgcaa cggagcgttt gcgtcaactt catcaacagc accatccgcg ggcataa 897

<210> 25
<211> 4032
<212> DNA
<213> Salmonella typhi

<400> 25
ttgagccagg aatatactga agacaaagac gtcacattga cgaagttaag cagcgggcgc 60

cgacttctgg aagcgttgtt gatacttatt gccctttttg ccgtctggtt gatggcggcc 120

ttgttgagct ttaacccttc ggaccccagt tggtcgcaaa ccgcctggca tgaacccatc 180

cataatttag gcggcgcgcc gggcgcgtgg ctggccgata cactgttctt tatttttggc 240

gtaatggcct acactatccc tgtcattatt gtcggcgggt gttggtttgc gtggcgtcat 300

cagtctacgg atgattacat cgactatttt gctgtgtcgc tacgccttat tggcgtgctg 360

gcgctgattc tcacttcctg cggtctggcg gccattaatg ccgacgatat ctggtatttc 420

gcctccggcg gcgtaattgg cagcctgtta agcaccacgc tgcagccgtt gttgcatagt 480

agcggcggaa cgataatgct tctctgtatt tgggctgccg ggctgacgct ctttaccggc 540

tggtcctggg ttagtatcgc cgaaaaatta ggcggctggc tgcttaatat actgaccttc 600

gccagcaacc gtacccggcg tgatgatacg tgggttgacg acgaagagta tgatgacgaa 660

tacgatgaag aaaccgatgg cgtgcagcgt gaatctcgcc gggcgcgtat tttgcgcggc 720

gcgttggcgc gtcgtaagcg actggctgaa aaattcagca atccacgggg ccgtcagacc 780

gatgccgcgc tcttttccgg caaacggatg gatgatgacg aggacattca gtatagcgcg 840

cgcggcgtcg ctgccgatcc cgacgacgtt ctgttttcgg gcaaccgtgc gacgcagtcg 900

gaatatgatg aatatgatcc gctgttaaac ggccattctg tcaccgaacc ggtcgccgcc 960

gctgcggccg caactgccgt cacccagacg tgggcagcgt ccgccgatcc cataatgcaa 1020

acgccgccca tgccgggggc tgagccggtt gtcgcgcaac cgaccgtgga gtggcagccc 1080

gtaccggggc cgcaaaccgg cgaaccggtt atcgcgcctg cgccggaggg gtatcagccg 1140

catccgcaat atgctcaacc gcaggaggcg caaagtgcgc cgtggcagca acctgtgcct 1200

gtcgcttccg cgccgcaata cgccgcgacg ccagcgaccg cagcagaata tgactctctt 1260

gcgccgcagg aaacacagcc gcaatggcag gcgccggatg ccgaacaaca ctggcagccg 1320

gagccgatag cggctgaacc ttcccacatg ccgccgccgg ttatcgaaca gccggttgcc 1380

accgagcctg aacctggcat agaagagacc aggcccgctc gccctccgct ttactatttt 1440

gaagaggtgg aagagaaacg cgcccgtgag cgcgaacaac tggcggcatg gtatcaaccg 1500

attccggagc cggtaaaaga gaacgtgccg gtcaaaccta cggtatctgt cgcgccgtcc 1560

ataccgccag tggaagcggt agccgccgcc gcgtcgcttg acacaggtat taagagtggc 1620

gccctggcag caggcgccgc ggccgctgcg cctgcattta gtctggctac cggcggcgcg 1680

ccgcgtccgc aggtgaaaga ggggattggt ccgcaactgc cgcgtccgaa ccgcgttcgc 1740

gtgccgaccc gacgggaact ggcatcgtac ggtattaagc tgccgtcaca gaggattgct 1800

gaagaaaaag cccgcgaggc ggagcgcaat cagtacgaga cgggcgcgca gctaaccgat 1860

gaagaaatag atgcaatgca tcaggacgag ttagcccgtc agtttgcgca gtcgcagcag 1920

catcgttatg gtgagacgta tcagcatgat actcaacagg cagaagatga cgataccgct 1980

gcggaagctg aattagcccg ccagtttgcc gcttcgcaac agcagcgcta ttccggcgag 2040

caacccgctg gcgcgcagcc gttctcactg gacgatctgg atttctcgcc aatgaaagta 2100

ttggttgatg aaggtccgca cgaaccgctg ttcacgccgg gtgtcatgcc cgagtcaacg 2160

cctgttcagc aacctgtagc gccgcagccg cagtatcagc agccggtagc gccgcagcca 2220

cagtatcagc agccgcagca gccggtagcg tcgcagccac agtatcagca gccgcaacag 2280

ccggtagcgc ctcagccgca gtatcagcag ccgcagcagc cggtagcgcc gcagccgcag 2340

tatcagcagc cgcaacagcc ggtagcgccg cagccgcagt atcagcaacc gcaacagccg 2400

gtagcgccgc agccacagta tcagcagccg caacagccgg tagcgcctca gccgcagtac 2460

cagcagccgc agcaaccgac agcgccgcaa gacagtctga tccacccgct gttgatgcgt 2520

aacggcgaca gtcgtccatt gcagcgtccg acgacgccgc tgccttcatt agatcttctg 2580

acgccgccgc cgagcgaagt tgaaccggta gatacctttg cgctggaaca aatggcgcgt 2640

ctggttgaag cgcgtctggc ggatttccgc attaaagctg atgtggtgaa ctattcgcca 2700

ggcccggtca ttactcgctt tgaactgaat ctggccccgg gcgttaaagc agcgcgtatc 2760

tccaaccttt cccgggatct ggcgcgctcc ttgtcaacgg tagccgtacg cgttgtggaa 2820

gtcattccag gcaaacctta cgtagggttg gaattgccga acaagaaacg gcaaaccgtt 2880

tatctgcgcg aagtgttgga taacgccaaa ttccgcgaga atccgtcgcc gcttaccgtg 2940

gtgttaggta aggatatcgc gggcgatccg gtagtggcgg atctggcgaa aatgcctcac 3000

ctgctggtgg ccggtacgac cggttccggt aagtcggtcg gggttaacgc catgatcctc 3060

agtatgctgt ataaagcgca gccggaagac gttcgcttta tcatgatcga cccgaaaatg 3120

ttagaactct cggtctatga aggtataccg catctgctga cggaagttgt taccgatatg 3180

aaagacgcag ctaacgcgtt acgctggagc gtcaatgaga tggagcgtcg ctataagctg 3240

atgtccgcgc tcggcgtgcg taatctggct ggttataacg agaaaatcgc cgaagcggcc 3300

cgcatgggac gtccgattcc ggacccgtac tggaagccgg gcgacagtat ggatgttcag 3360

catccggttc tggaaaaact gccgtatatc gtggtactgg ttgatgaatt tgccgacctg 3420

atgatgaccg tcggtaaaaa agttgaagag ctgatcgcgc gtttggcgca gaaagcgcgc 3480

gctgcgggga ttcacctggt actggcgacg cagcgtccgt cggtcgatgt gattactggt 3540

ttaattaaag ccaacattcc aacccgtatc gcttttacag tatccagcaa gatcgactcc 3600

cgcaccattc ttgatcaggg tggcgctgag tcgttattgg gaatggggga catgctctat 3660

tccggaccaa actccaccat gccggtacgt gtccatgggg cgtttgtgcg tgaccaggaa 3720

gtccatgcgg tggtgcagga ctggaaagcg cgcggtcgtc cgcaatatgt ggatggcatt 3780

acctccgaca gcgaaagcga aggtggcggt ggcggttttg acggcggcga agaattagat 3840

gcgctattcg atcaggccgt taatttcgtc actcagaagc gaaaagcgtc catttccggc 3900

gttcagcgtc agttccgtat cggctataac cgtgcggcgc gtattatcga acagatggaa 3960

gcgcagggta ttgtcagcgc tcaggggcat aacggtaatc gcgaagtgtt ggctccgccg 4020

ccttttgagt aa 4032

<210> 26
<211> 1032
<212> DNA
<213> Bacteriophage P1

<400> 26
atgtccaatt tactgaccgt acaccaaaat ttgcctgcat taccggtcga tgcaacgagt 60

gatgaggttc gcaagaacct gatggacatg ttcagggatc gccaggcgtt ttctgagcat 120

acctggaaaa tgcttctgtc cgtttgccgg tcgtgggcgg catggtgcaa gttgaataac 180

cggaaatggt ttcccgcaga acctgaagat gttcgcgatt atcttctata tcttcaggcg 240

cgcggtctgg cagtaaaaac tatccagcaa catttgggcc agctaaacat gcttcatcgt 300

cggtccgggc tgccacgacc aagtgacagc aatgctgttt cactggttat gcggcggatc 360

cgaaaagaaa acgttgatgc cggtgaacgt gcaaaacagg ctctagcgtt cgaacgcact 420

gatttcgacc aggttcgttc actcatggaa aatagcgatc gctgccagga tatacgtaat 480

ctggcatttc tggggattgc ttataacacc ctgttacgta tagccgaaat tgccaggatc 540

agggttaaag atatctcacg tactgacggt gggagaatgt taatccatat tggcagaacg 600

aaaacgctgg ttagcaccgc aggtgtagag aaggcactta gcctgggggt aactaaactg 660

gtcgagcgat ggatttccgt ctctggtgta gctgatgatc cgaataacta cctgttttgc 720

cgggtcagaa aaaatggtgt tgccgcgcca tctgccacca gccagctatc aactcgcgcc 780

ctggaaggga tttttgaagc aactcatcga ttgatttacg gcgctaagga tgactctggt 840

cagagatacc tggcctggtc tggacacagt gcccgtgtcg gagccgcgcg agatatggcc 900

cgcgctggag tttcaatacc ggagatcatg caagctggtg gctggaccaa tgtaaatatt 960

gtcatgaact atatccgtaa cctggatagt gaaacagggg caatggtgcg cctgctggaa 1020

gatggcgatt aa 1032

<210> 27
<211> 34
<212> DNA
<213> Bacteriophage P1

<400> 27
ataacttcgt ataatgtatg ctatacgaag ttat 34

<210> 28
<211> 1272
<212> DNA
<213> Saccharomyces cerevisiae

<400> 28
atgccgcaat ttgatattct atgcaaaacc cccccaaaag tgctggtgcg ccaatttgtg 60

gaacgatttg aacgcccgag cggcgagaaa attgcgttgt gtgctgcgga gcttacgtac 120

ctctgctgga tgattaccca caatggcacg gcgatcaaac gggcaacttt catgtcctat 180

aatacaatca tttcaaactc cttatccttt gacatcgtga acaagtccct gcaattcaaa 240

tataaaacgc agaaagcgac catcctggaa gcgagcctga aaaaacttat ccctgcgtgg 300

gagtttacca tcatccccta ttacggccag aagcatcagt cagacatcac cgatattgtg 360

agcagtctcc agcttcagtt cgagtcgagc gaagaagcag ataagggaaa cagtcacagc 420

aaaaaaatgc tgaaggcgct tctgagcgag ggagagtcga tttgggaaat aactgaaaaa 480

attttgaaca gtttcgagta taccagccgg tttacaaaga cgaaaaccct ttatcaattc 540

ctgtttctgg cgacctttat taactgcggg cgtttttccg acatcaaaaa tgttgacccg 600

aaatcattta aactggtcca gaataaatat ctgggtgtga ttatacagtg ccttgttaca 660

gaaaccaaaa cgagcgttag ccgtcatatc tactttttta gtgcaagagg tcgtattgat 720

ccgctggtat atctggatga atttcttcgt aatagcgagc cagtcctcaa gcgcgtgaac 780

agaaccggca acagcagctc caacaaacag gaatatcagc tgctgaagga caacttagta 840

cgctcttaca ataaggctct gaaaaaaaac gcaccatatt ccatctttgc aatcaaaaat 900

ggccccaaaa gccacattgg gcgacatctg atgacgtcct tcctgtccat gaaaggttta 960

actgagctga ccaatgtagt tggcaattgg tctgacaaac gtgcatccgc tgtcgcccgc 1020

acgacctaca cccaccagat cacggcgata ccggatcatt attttgccct ggtgtcccgc 1080

tattatgcgt atgatcccat ttctaaagaa atgattgcct taaaagatga aactaacccg 1140

atcgaagagt ggcagcatat cgaacagctg aaagggagcg cggaaggcag tattcgctac 1200

ccggcctgga atgggattat ctcgcaggaa gttctggact acctgtcttc ttacattaac 1260

cgtcggatct aa 1272

<210> 29
<211> 34
<212> DNA
<213> Saccharomyces cerevisiae

<400> 29
gaagttccta ttctctagaa agtataggaa cttc 34

<210> 30
<211> 493
<212> DNA
<213> Bacteriophage lambda

<400> 30
acgttaaatc tatcaccgca agggataaat atctaacacc gtgcgtgttg actattttac 60

ctctggcggt gataatggtt gcatgtacta aggaggttgt atggaacaac gcataaccct 120

gaaagattat gcaatgcgct ttgggcaaac caagacagct aaagatctct cacctaccaa 180

acaatgcccc cctgcaaaaa ataaattcat ataaaaaaca tacagataac catctgcggt 240

gataaattat ctctggcggt gttgacataa ataccactgg cggtgatact gagcacatca 300

gcaggacgca ctgaccacca tgaaggtgac gctcttaaaa attaagccct gaagaagggc 360

agcattcaaa gcagaaggct ttggggtgtg tgatacgaaa cgaagcattg ggatctatcg 420

atgcatgcca tggtacccgg gagctcgaat taattctaga aataattttg tttaacttta 480

agaaggagat ata 493

<210> 31
<211> 714
<212> DNA
<213> Bacteriophage lambda

<400> 31

atgagcacaa aaaagaaacc attaacacaa gagcagcttg aggacgcacg tcgccttaaa 60

gcaatttatg aaaaaaagaa aaatgaactt ggcttatccc aggaatctgt cgcagacaag 120

atggggatgg ggcagtcagg cgttggtgct ttatttaatg gcatcaatgc attaaatgct 180

tataacgccg cattgcttgc aaaaattctc aaagttagcg ttgaagaatt tagcccttca 240

atcgccagag aaatctacga gatgtatgaa gcggttagta tgcagccgtc acttagaagt 300

gagtatgagt accctgtttt ttctcatgtt caggcaggga tgttctcacc taagcttaga 360

acctttacca aaggtgatgc ggagagatgg gtaagcacaa ccaaaaaagc cagtgattct 420

gcattctggc ttgaggttga aggtaattcc atgaccgcac caacaggctc caagccaagc 480

tttcctgacg gaatgttaat tctcgttgac cctgagcagg ctgttgagcc aggtgatttc 540

tgcatagcca gacttggggg tgatgagttt accttcaaga aactgatcag ggatagcggt 600

caggtgtttt tacaaccact aaacccacag tacccaatga tcccatgcaa tgagagttgt 660

tccgttgtgg ggaaagttat cgctagtcag tggcctgaag agacgtttgg ctaa 714

<210> 32
<211> 338
<212> DNA
<213> Escherichia coli

<400> 32
cgtttcactc catccaaaaa aacgggtatg gagaaacagt agagagttgc gataaaaagc 60

gtcaggtagg atccgctaat cttatggata aaaatgctat ggcatagcaa agtgtgacgc 120

cgtgcaaata atcaatgtgg acttttctgc cgtgattata gacacttttg ttacgcgttt 180

ttgtcatggc tttggtcccg ctttgttaca gaatgctttt aataagcggg gttaccggtt 240

gggttagcga gaagagccag taaaagacgc agtgacggca atgtctgatg caatatggac 300

aattggtttc ttctctgaat ggtgggagta tgaaaagt 338

<210> 33
<211> 879
<212> DNA
<213> Escherichia coli

<400> 33
atggctgaag cgcaaaatga tcccctgctg ccgggatact cgtttaacgc ccatctggtg 60

gcgggtttaa cgccgattga ggccaacggt tatctcgatt tttttatcga ccgaccgctg 120

ggaatgaaag gttatattct caatctcacc attcgcggtc agggggtggt gaaaaatcag 180

ggacgagaat ttgtctgccg accgggtgat attttgctgt tcccgccagg agagattcat 240

cactacggtc gtcatccgga ggctcgcgaa tggtatcacc agtgggttta ctttcgtccg 300

cgcgcctact ggcatgaatg gcttaactgg ccgtcaatat ttgccaatac gggtttcttt 360

cgcccggatg aagcgcacca gccgcatttc agcgacctgt ttgggcaaat cattaacgcc 420

gggcaagggg aagggcgcta ttcggagctg ctggcgataa atctgcttga gcaattgtta 480

ctgcggcgca tggaagcgat taacgagtcg ctccatccac cgatggataa tcgggtacgc 540

gaggcttgtc agtacatcag cgatcacctg gcagacagca attttgatat cgccagcgtc 600

gcacagcatg tttgcttgtc gccgtcgcgt ctgtcacatc ttttccgcca gcagttaggg 660

attagcgtct taagctggcg cgaggaccaa cgcattagtc aggcgaagct gcttttgagc 720

actacccgga tgcctatcgc caccgtcggt cgcaatgttg gttttgacga tcaactctat 780

ttctcgcgag tatttaaaaa atgcaccggg gccagcccga gcgagtttcg tgccggttgt 840

gaagaaaaag tgaatgatgt agccgtcaag ttgtcataa 879

<210> 34
<211> 81
<212> DNA
<213> Escherichia coli

<400> 34
cattaattcc taatttttgt tgacactcta tcattgatag agttatttta ccactcccta 60

tcagtgatag agaaaagtga a 81

<210> 35
<211> 624
<212> DNA
<213> Escherichia coli

<400> 35
atgtctagat tagataaaag taaagtgatt aacagcgcat tagagctgct taatgaggtc 60

ggaatcgaag gtttaacaac ccgtaaactc gcccagaagc taggtgtaga gcagcctaca 120

ttgtattggc atgtaaaaaa taagcgggct ttgctcgacg ccttagccat tgagatgtta 180

gataggcacc atactcactt ttgcccttta gaaggggaaa gctggcaaga ttttttacgt 240

aataacgcta aaagttttag atgtgcttta ctaagtcatc gcgatggagc aaaagtacat 300

ttaggtacac ggcctacaga aaaacagtat gaaactctcg aaaatcaatt agccttttta 360

tgccaacaag gtttttcact agagaatgca ttatatgcac tcagcgctgt ggggcatttt 420

actttaggtt gcgtattgga agatcaagag catcaagtcg ctaaagaaga aagggaaaca 480

cctactactg atagtatgcc gccattatta cgacaagcta tcgaattatt tgatcaccaa 540

ggtgcagagc cagccttctt attcggcctt gaattgatca tatgcggatt agaaaaacaa 600

cttaaatgtg aaagtgggtc ttaa 624

<210> 36
<211> 466
<212> DNA
<213> Salmonella typhimurium

<400>  36
tattgccatc gcggatgtcg cctgtcttat ctaccatcat aaacatcatt tgcctatggc      60

tcacgacagt ataggcaatg ccgtttttta tattgctaat tgtttcgcca atcaacgcaa     120

aagtatggcg attgctaaag ccgtctccct gggcggtaga ttagccttaa ccgcgacggt     180

aatgactcat tcatactgga gtggtagttt gggactacag cctcatttat tagagcgtct     240

taatgatatt acctatggac taatgagttt tactcgcttc ggtatggatg ggatggcaat     300

gaccggtatg caggtcagca gcccattata tcgtttgctg gctcaggtaa cgccagaaca     360

acgtgcgccg gagtaatcgt tttcaggtat ataccggatg ttcattgctt tctaaatttt     420

gctatgttgc cagtatcctt acgatgtatt tattttaagg aaaagc                    466

<210>  37
<211>  2046
<212>  DNA
<213>  Salmonella enterica

<400>  37
atgaggtcgt ggctcggcga gggcgtgcgc gcccaacaat ggctgagcgt gtgcgctgga      60

cgccaggaca tggtcctggc taccgtcctg ctcatcgcaa tcgtcatgat gctcctgcct     120

ctgccaacct ggatggtgga catcctcatt acgattaatc taatgttctc ggtcatcctt     180

ctcctgattg caatctacct gagcgacccg ctggacctct cagtcttccc tagcctgctc     240

ctgattacca cgctgtaccg gctgtcgcta accatctcca cctcccgctt ggtgctgctc     300

cagcacaacg caggcaatat cgtggacgca tttggaaagt tcgtggtggg aggaaacctg     360

accgtcggac tggtggtctt cactatcatc accattgtcc agttcatcgt gattactaaa     420

ggcattgagc gggtggctga ggtgtcagcc cggttctccc tggatggtat gcccggaaag     480

caaatgtcga tcgacggaga tctacgcgcc ggcgtcatcg acgcggacca tgcgcggacc     540

ctccgccaac acgtccaaca ggaatcacgc ttcctcggtg caatggacgg agccatgaaa     600

ttcgtgaagg gagacaccat cgcagggatc attgtggtcc tggtcaacat cattggcggc     660

attatcatcg ccattgtcca gtacgatatg tcaatgtccg aagcagtcca cacctactcg     720

gtcctgagca tcggcgacgg actctgcggt cagatcccta gcttgcttat ctcactgtcc     780

gccggaatca tcgtgactcg cgtcccaggc gaaaagcggc agaatctcgc caccgaactc     840

tcatcgcaga tcgcgcgcca gccccagagc ctgattctga ctgcggtcgt gctgatgctg     900

ctggccctca tccccggttt cccgtttatc accctggcat ttttcagcgc tctgctcgca     960

ctaccgatca tcctgatccg cagaaagaag tccgtcgtgt cggcaaatgg agtggaagcc    1020

ccagaaaagg actcgatggt cccaggagct tgcccactga tcctgcggct cagccctacc    1080

ctgcattcgg cggacctcat ccgggacatc gacgcgatgc gctggttcct gttcgaggat    1140

acgggagtgc ctctgcctga agtgaatatc gaagtcttgc cagaacctac tgagaagctc    1200

acggtgctgc tgtaccaaga accggtcttc tcactgtcga ttccagctca ggctgattac 1260

ctcctgattg gagcagacgc ttcagtcgtg ggagactccc agactctgcc gaatggcatg 1320

ggccaaatct gctggctgac taaggatatg gcacataaag cacagggatt cggcctagac 1380

gtgtttgccg gatcgcagag gattagcgcg ctgctgaagt gcgtcctgct gcgccacatg 1440

ggcgagttca ttggggtcca ggagacccgg tatctgatga atgcaatgga gaagaactac 1500

tccgaactgg tcaaggaact acagcggcag ttgccgatta acaagatcgc cgagactctg 1560

cagcggctcg tgagcgaaag agtgtcaatc cgggatcttc ggctgatctt cgggactctg 1620

atcgactggg ctccgcggga gaaagacgtc ctcatgctga ctgagtacgt gagaatcgcc 1680

ctccggagac acatcctccg cagactgaac cccgagggga agccgttgcc tatcctcagg 1740

atcggagagg gtatcgaaaa tctggtccgg gagtcgatcc ggcagaccgc gatgggaacc 1800

tacaccgccc tctccagccg ccataaaacc cagatcctcc agctgatcga acaagcgctc 1860

aagcagagcg cgaagctgtt catcgtgacc tcggtggaca cgagaaggtt cctgcggaag 1920

atcactgagg cgaccctgtt cgatgtccct attctgtcgt ggcaggagct gggcgaagag 1980

agcctcatcc aggtcgtgga atccatcgac ttgtcggaag aagaactcgc cgacaacgag 2040

gaataa 2046

<210> 38
<211> 2129
<212> DNA
<213> Salmonella typhimurium

<400> 38
gaagttccta ttctctagaa agtataggaa cttcttgtgt ctcaaaatct cttccctttt 60

taacaaggaa tttttatggc acgcattatt gttgttactt cgggtaaagg gggcgttggc 120

aagaccacct ccagcgcggc catcgctaca ggtttggccc agaagggaaa gaaaactgtc 180

gtcattgatt ttgatatcgg actgcgtaac ctcgatctga ttatggggtg cgaacgtcgt 240

gtcgtttacg attttgtaaa cgtcattcag ggcgatgcga cactgaatca ggcgctgatc 300

aaagataagc gtactgaaaa tctcttcatt cttccggcgt cgcagacccg ggataaagac 360

gcgctaacgc gcgaaggcgt cgctaaggta ctggactcac tgaaagcgat ggactttgag 420

ttcatcgttt gcgactcgcc ggcgggtatc gaaaccgggg cgctgatggc gctctatttt 480

gccgatgaag cgatcatcac gaccaacccg gaagtctctt ctgtccgtga ctcggaccgt 540

attctgggta ttctggcatc gaaatctcgt cgcgcagaaa atggcgaaga accgattaaa 600

gaacatctcc tgttgacgcg ctacaatcca ggccgcgtca ataaaggcga catgctcagc 660

atggaagatg tactggagat tctgcgtatt aaactcgtcg gtgtgatccc ggaagatcaa 720

tccgtactgc gcgcatcgaa ccagggcgaa ccggtgattc ttgacgccac tgcggatgcg 780

ggtaaagcct atgcagatac cgtagatcgt ctgttgggag aagaacgtcc tttccgcttc 840

attgaagaag agaagaaagg tttcctcaaa cgcctgttcg gaggataaat tatggcatta 900

ctggattttt ttctctcgcg gaaaaagagt accgctaata ttgcaaaaga gcggttgcaa 960

atcattgtcg cggaacgtcg ccgtagcgat gccgaaccgc attatttacc gcagttacgt 1020

aaagatattc ttgaggtcat ttgtaaatac gtacaaatcg atccggaaat ggttaccgtc 1080

cagcttgagc aaaaagatgg tgatatctcc atccttgaac ttaacgtaac gctgccggaa 1140

gccgaagagt caaaataagc tgtgtctcaa aatctctgat gttacattgc acaagataaa 1200

aatatatcat catgaacaat aaaactgtct gcttacataa acagtaatac aaggggtgtt 1260

atgagccata ttcaacggga aacgtcttgc tcgaggccgc gattaaattc caacatggat 1320

gctgatttat atgggtataa atgggctcgc gataatgtcg ggcaatcagg tgcgacaatc 1380

tatcgattgt atgggaagcc cgatgcgcca gagttgtttc tgaaacatgg caaaggtagc 1440

gttgccaatg atgttacaga tgagatggtc agactaaact ggctgacgga atttatgcct 1500

cttccgacca tcaagcattt tatccgtact cctgatgatg catggttact caccactgcg 1560

atccccggga aaacagcatt ccaggtatta gaagaatatc ctgattcagg tgaaaatatt 1620

gttgatgcgc tggcagtgtt cctgcgccgg ttgcattcga ttcctgtttg taattgtcct 1680

tttaacagcg atcgcgtatt tcgtctcgct caggcgcaat cacgaatgaa taacggtttg 1740

gttgatgcga gtgattttga tgacgagcgt aatggctggc ctgttgaaca agtctggaaa 1800

gaaatgcata agcttttgcc attctcaccg gattcagtcg tcactcatgg tgatttctca 1860

cttgataacc ttatttttga cgaggggaaa ttaataggtt gtattgatgt tggacgagtc 1920

ggaatcgcag accgatacca ggatcttgcc atcctatgga actgcctcgg tgagttttct 1980

ccttcattac agaaacggct ttttcaaaaa tatggtattg ataatcctga tatgaataaa 2040

ttgcagtttc atttgatgct cgatgagttt ttctaatcag aattggttaa ttggtgaagt 2100

tcctatactt tctagagaat aggaacttc 2129

<210> 39
<211> 102
<212> DNA
<213> Salmonella typhimurium

<400> 39
gaagttccta ttctctagaa agtataggaa cttcttgtgt ctcaaaatct cttccctttt 60

taacaaggaa tttttatgtc ttttatggga aaattcctgg at 102

<210> 40
<211> 681
<212> DNA
<213> Artificial Sequence

<220>  Synthetic RFP Sequence
<223>  Synthetic RFP Sequence

<400>  40
atggcaagca gcgaagatgt gatcaaagaa tttatgcgtt tcaaagtgcg tatggaaggt      60

agcgttaatg gccatgaatt tgaaattgaa ggtgaaggcg aaggtcgtcc gtatgaaggc     120

acccagaccg caaaactgaa agttaccaaa ggtggtccgc tgccgtttgc atgggatatt     180

ctgagtccgc agtttcagta tggtagcaaa gcctatgtta aacatccggc agatatcccg     240

gattatctga aactgagctt tccggaaggt tttaaatggg aacgtgtgat gaattttgaa     300

gatggtggtg ttgttaccgt tacccaggat agcagcctgc aggatggtga atttatctat     360

aaagttaaac tgcgtggcac caattttccg agtgatggtc cggttatgca gaaaaaaacc     420

atgggttggg aagcaagcac cgaacgtatg tatccggaag atggcgcact gaaaggtgaa     480

atcaaaatgc gcctgaaact gaaagatggt ggccattatg atgcagaagt taaaaccacc     540

tacatggcca aaaaaccggt tcagctgcct ggtgcatata aaaccgatat taaactggat     600

atcaccagcc acaacgagga ttataccatt gttgaacagt atgaacgtgc agaaggtcgc     660

catagtaccg gtgcataata a                                               681


<210>  41
<211>  30
<212>  DNA
<213>  Salmonella typhi

<400>  41
aatcatatgt ttgaacctat ggaactaacc                                       30


<210> 42
<211> 38
<212> DNA
<213> Salmonella typhi

<400> 42
aatctagaat tatccagtgg caacaacggt cacgcgca                              38


<210> 43
<211> 30
<212> DNA
<213> Salmonella typhi

<400> 43
aatcatatgc agtacccgat taacgagatg                                       30


<210> 44
<211> 36
<212> DNA
<213> Salmonella typhi

<400> 44
aaagtcgaca ttaggcgata tttaaatatt tatgcg                                36

## Description

### Field of the Invention

The invention relates to genetically modified bacterial vaccine carriers, methods of producing the same and their uses in the treatment or prevention of disease.

### Background of the Invention

Live bacterial vectors (LBVs) have been used extensively in experiments to express and deliver recombinant protein vaccines and anti-cancer compounds to humans and other animals. LBVs are usually constructed by introducing into a host bacterium an expression cassette containing a promoter which regulates the expression of one or more genes encoding heterologous antigens from one or more pathogens. This cassette can either be chromosomally integrated in the host bacterial genome or carried by the host as an autonomously replicating multi-copy plasmid (Kochi et al. 2003, Expert Rev. Vaccines 2:31-43). Post-delivery of the LBV to a subject to be treated, the expressed heterologous antigen is delivered and presented to the subject's immune system to promote a prophylactic or therapeutically effective immune response.

LBVs are usually delivered orally for vaccine applications, either in a liquid form as an oral suspension or as an enterically coated capsule. The LBVs then enter the small intestine where they invade the intestinal epithelium via microfold (M) cells into Peyer's patches from where they are subsequently delivered to antigen-presenting cells (APCs) (Kotton & Hohmann 2004, Infect. Immun. 72: 5535-5547). Alternative delivery methods are available, and indeed often used, for example in anti-cancer applications, where the LBV may be injected systemically (e.g. intravenously) or directly into a tumour.

The standard approach for preparing LBVs from a host bacterium is to introduce specific, attenuating mutations into pathogenic genes of the host bacterium. These attenuating mutations reduce the survival of the host bacterium within the subject to prevent LBV-mediated side-effects.

The field has numerous examples of animal studies using host bacteria comprising attenuating genetic mutations. In general, these LBVs are sufficient to induce a prophylactic response against the heterologous antigen, but are not safe enough to be used in humans. This provides a false picture of the potential utility of LBVs for recombinant vaccine delivery.

Most commonly, attenuating mutations are made in genes involved in the aromatic amino acid (shikimate) biosynthesis pathway, such as *aroA* and *aroC.* However, although such attenuated LBVs are well tolerated in mice, these cause undesirable feverish symptoms and prolonged bacterial shedding in humans (Garmory et al. 2002, FEMS Microbiol. Rev. 26: 339-353). Conversely when a host bacterium is sufficiently attenuated for human use to deliver a heterologous antigen, the efficacy of the LBV is invariably disappointing (Garmory et al. 2002, FEMS Microbiol. Rev. 26: 339-353). This is because an improved LBV safety profile correlates with reduced immunogenicity. For example, extensive transposon-mediated insertional mutagenesis of the *Salmonella enterica* serovar Typhimurium genome failed to identify a mutant that was sufficiently immunogenic yet unable to cause disease in immunocompromised mice (Grant et al. 2016, Infect. Immun. 84: 989-997).

Therefore, there is a need to develop new methods for generating LBVs to improve their safety profile but maintain sufficient immunogenicity to act as effective vaccine carriers.

### Summary of the Invention

The present invention is based on the surprising discovery that a host bacterium genetically modified to conditionally repress cell division can attenuate the bacterium under those conditions without simultaneously suppressing cell elongation or the ability of the bacterium to act as an effective vaccine carrier.

Thus, in a first aspect of the invention, there is provided a genetically engineered non-natural bacterium comprising one or more of the following genetic modifications: (i) one or more heterologous genes encoding factors that prevent cell division, the expression of which are under the control of an inducible promoter activated when the bacterium is internalised into the intracellular vesicle of an animal cell; (ii) one or more endogenous factors that promote cell division, the expression of which are genetically modified to be repressed when the bacterium is internalised into the intracellular vesicle of an animal cell; and/or (iii) one or more endogenous factors that repress cell division, the expression of which are genetically modified to be under the control of an inducible promoter activated when the bacterium is internalised into the intracellular vesicle of an animal cell.

In a second aspect of the invention, there is provided a non-natural bacterium according to the first aspect of the invention, for use in the prevention or treatment of diseases, including cancer.

In a third aspect of the invention, there is provided the use of the bacterium of the first aspect of the invention in the vaccination of a subject against a disease.

In a fourth aspect of the invention, there is provided a method of manufacturing a non-natural bacterium according to the first aspect of the invention comprising introducing into the bacterium any of the genetic modifications of the first aspect of the invention.

### Description of the Figures

The invention is illustrated by the following drawings, in which:

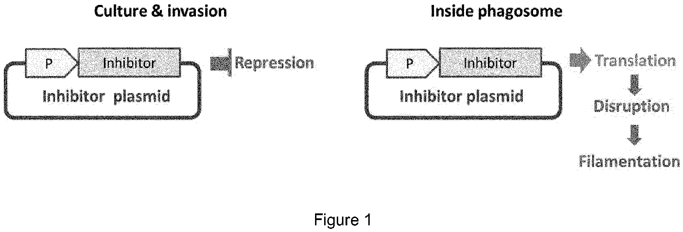

**Figure 1** shows a plasmid comprising a gene that inhibits cell division (inhibitor) under the control of a phagosomal promoter P. The gene is therefore not transcribed under standard laboratory conditions or during the initial stages of invasion of a subject to be treated. When the non-natural bacterium comprising the plasmid is internalised into a phagosome, the expression of the inhibitor is activated to prevent cell division and induce bacterial filamentation.

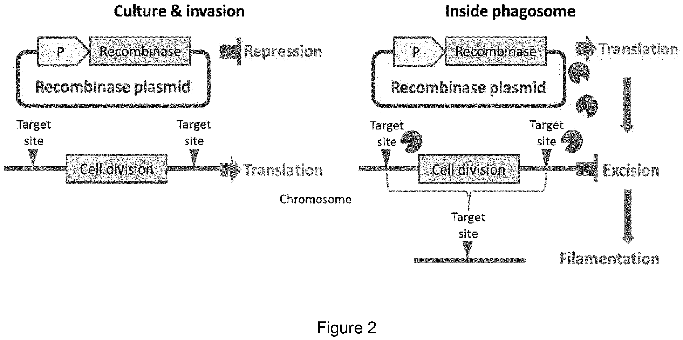

**Figure 2** shows a plasmid comprising a gene encoding a site-specific recombinase gene under the control of a phagosomal promoter P. The site specific recombination sites targeted and cleaved by the recombinase are engineered to flank a chromosomal gene encoding a factor essential for cell division. When the non-natural bacterium comprising the plasmid is internalised into a phagosome, the expression of the recombinase gene is activated, and the gene encoding the factor essential for cell division is excised, thereby inducing bacterial filamentation.

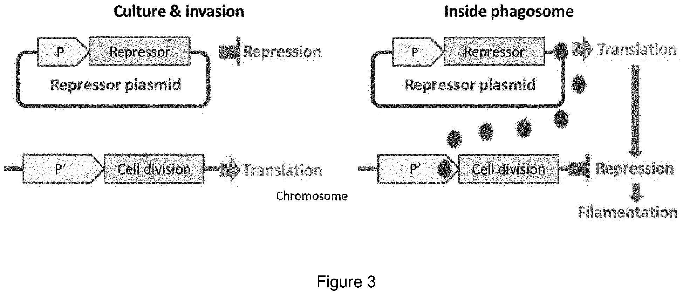

**Figure 3** shows a plasmid comprising a gene encoding a transcriptional repressor protein under the control of a phagosomal promoter P. The native promoter of a chromosomal gene encoding a factor essential for correct cell division is replaced with the cognate inducible promoter bound by the repressor protein. When the host bacterium comprising the plasmid is internalised into a phagosome, the expression of the repressor protein is activated, thus repressing expression of the gene essential for cell division, resulting in bacterial filamentation.

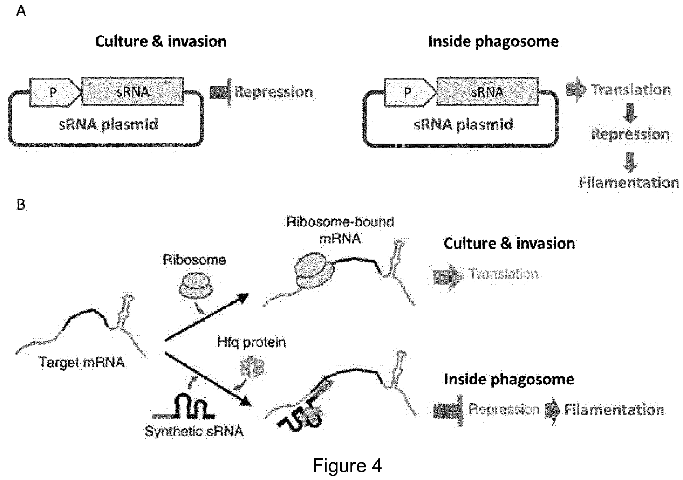

**Figure 4** shows A) a plasmid carrying a gene encoding a sRNA that binds to and represses the expression of a mRNA encoding an endogenous factor essential for cell division. The sRNA gene is under the control of a phagosomal promoter P, and is thus only expressed when the host bacterium is internalised into a phagosome. B) The sRNA has a complementary antisense sequence to the 5' UTR of the target mRNA. The sRNA is bound and stabilised by the Hfq protein, which stabilises the binding of the sRNA to the target mRNA, thus blocking ribosome binding and expression of the target mRNA to prevent cell division, thereby inducing bacterial filamentation (adapted from Yoo et al. 2013, Nature Protocols 8: 1694-1707).

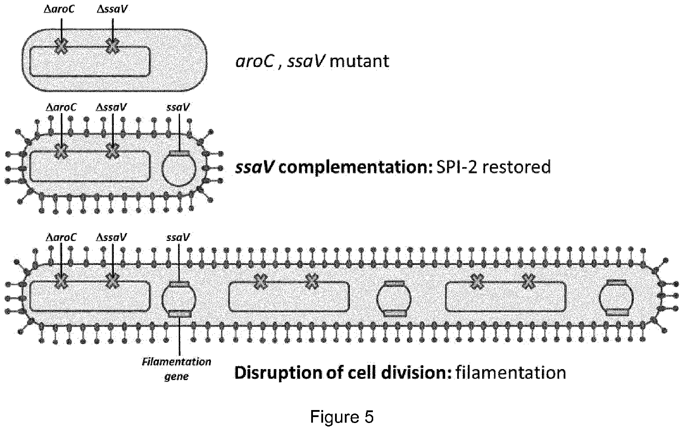

**Figure 5** shows a safety mechanism whereby the host bacterium is attenuated by the deletion of a chromosomally-encoded essential gene such as *aroC* or *ssaV.* The essential gene is complemented by the introduction of a heterologous functional copy of the gene on a plasmid, thus restoring the viability of the host. One of the genes described in Figures 1-3 is also cloned into the plasmid. Thus in the event of plasmid loss resulting in the loss of the filamentous phenotype, the heterologous essential gene is also lost, thus maintaining attenuation of the host bacterium.

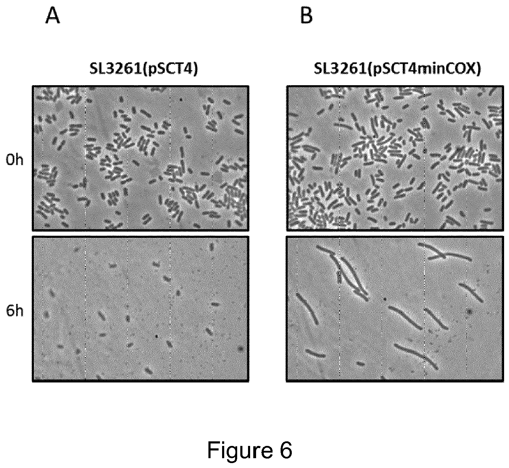

**Figure 6** shows A) *Salmonella* Typhimurium SL3261 containing the negative control plasmid pSCT4 (containing no genes) growing in PCN medium. After six hours of growth, the bacteria maintain the regular rod shape. B) S. Typhimurium SL3261 containing the plasmid pSCT4minCOX, with the P_{ssaG} promoter (SEQ ID NO: 35) controlling expression of the minC gene (SEQ ID NO: 1). The bacterial morphology was initially the same as the negative control strain, but after 6 hours of growth, minC over-expression arrested cell division and filamentous bacteria were produced.

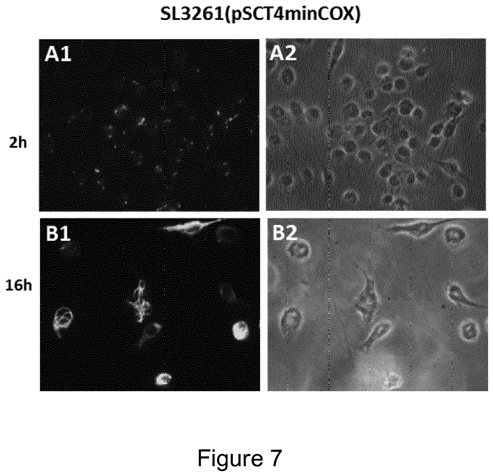

**Figure 7** shows S. Typhimurium SL3261 (pSCT4minCOX) infecting RAW264.7 macrophage cells under 1) immunofluorescence and 2) bright-field microscopy. A) The bacterial morphology was rod-shaped two hours post-infection. B) However, after sixteen hours of growth, MinC over-expression resulted in a filamentous bacterial phenotype.

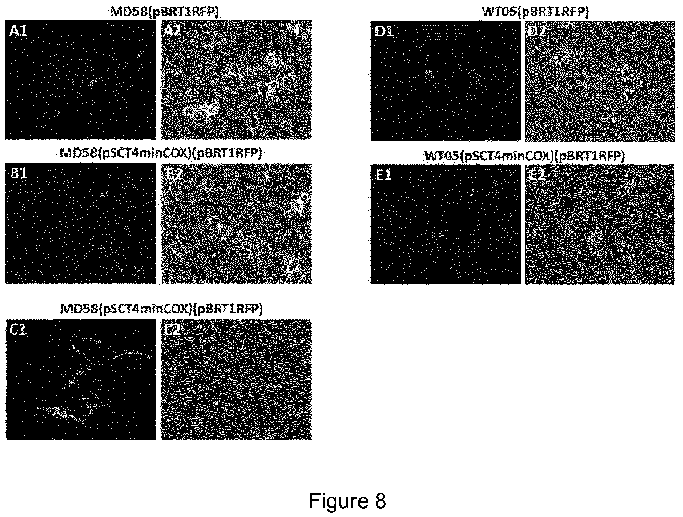

**Figure 8** shows S. Typhimurium strains containing plasmid pBRT1 RFP infecting RAW 264.7 cells at 16h post-infection under 1) fluorescence and 2) phase-contrast microscopy. Micrographs show A. MD58(pBRT1 RFP); B. MD58(pSCT4minCOX)(pBRT1 RFP); C. MD58(pSCT4minCOX)(pBRT1RFP) at x1000 magnification; D. WT05(pBRT1RFP); E. WT05(pSCT4minCOX)(pBRT1RFP). Cells containing pBRTIRFP retained their normal rod shape, but those that additionally contained pSCT4minCOX were filamentous.

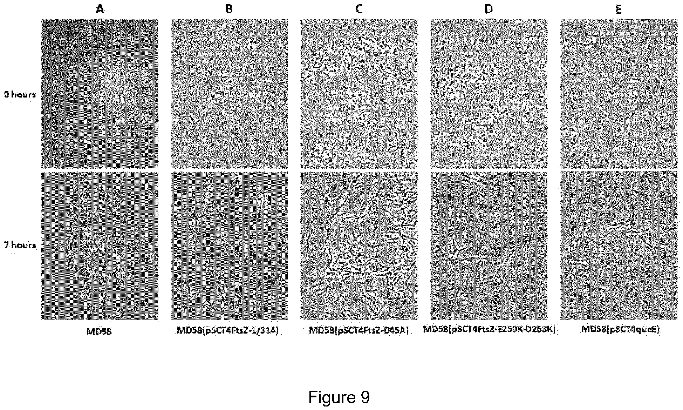

**Figure 9** shows phase contrast micrographs of bacterial cells grown for 7 hours in PCN medium. A. S. Typhimurium MD58 negative control strain with no plasmid; B. MD58(pSCT4FtsZ-1/314); C. MD58(pSCT4FtsZ-D45A); D. MD58(pSCT4FtsZ-E250K-D253K), E. MD58(pSCT4queE). Control strain MD58 maintains its rod-shaped morphology, whereas MD58 expressing mutants of FtsZ or QueE develop a filamentous phenotype.

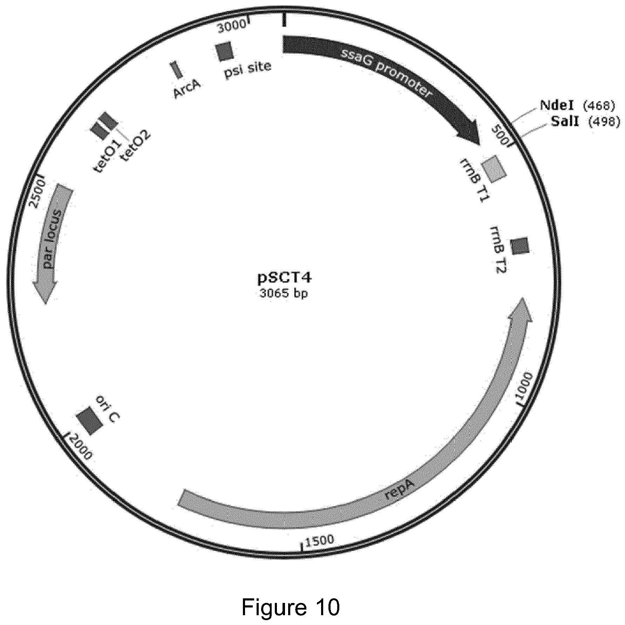

**Figure 10** shows a plasmid map of pSCT4

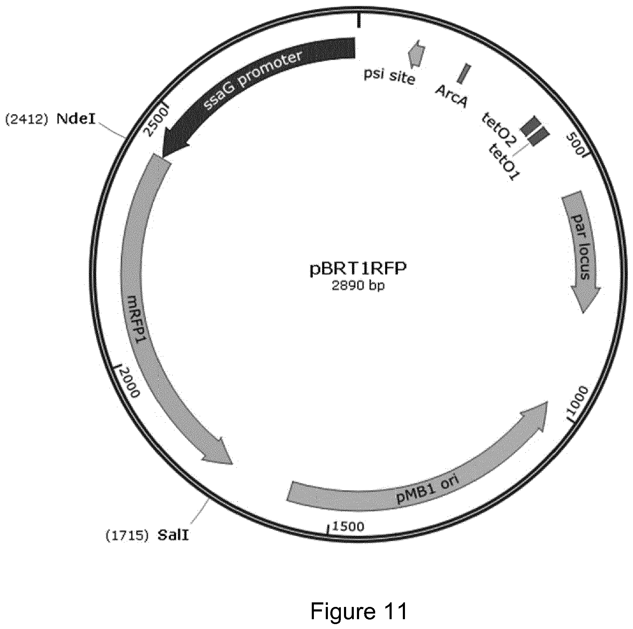

**Figure 11** shows a plasmid map of pBRT1 RFP

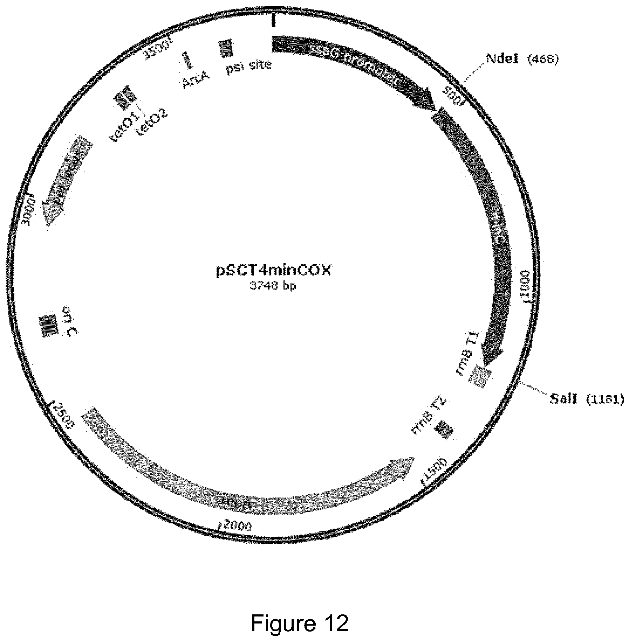

**Figure 12** shows a plasmid map of pSCT4minCOX

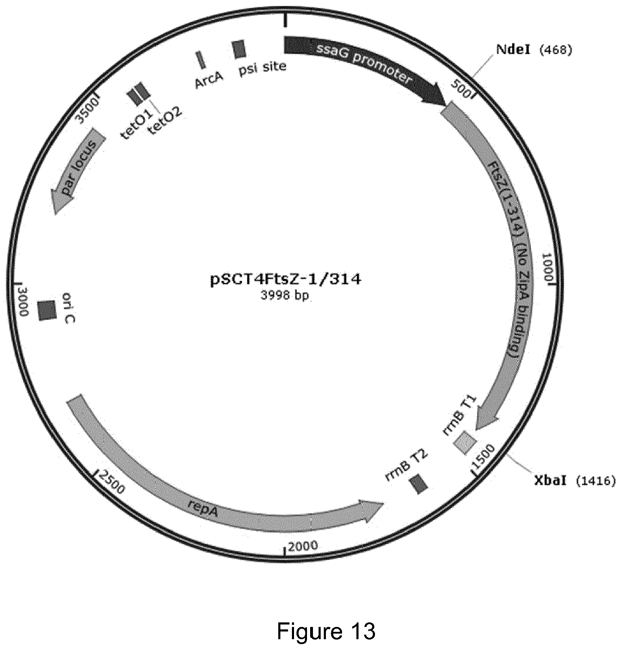

**Figure 13** shows a plasmid map of pSCT4FtsZ-1/314

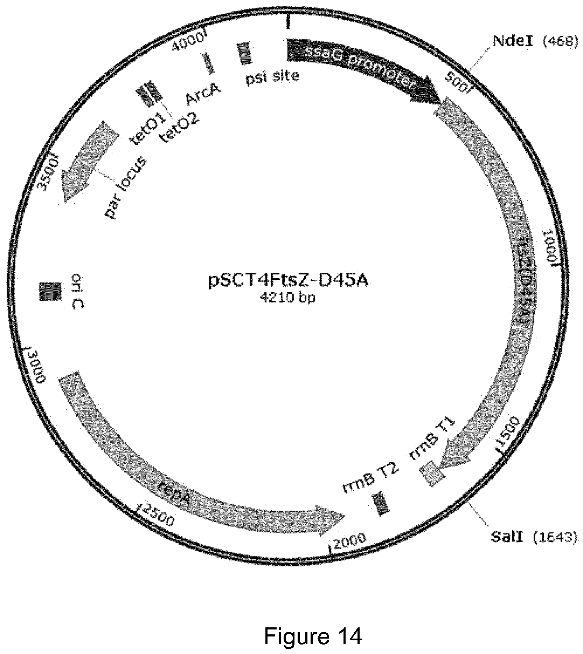

**Figure 14** shows a plasmid map of pSCT4FtsZ-D45A

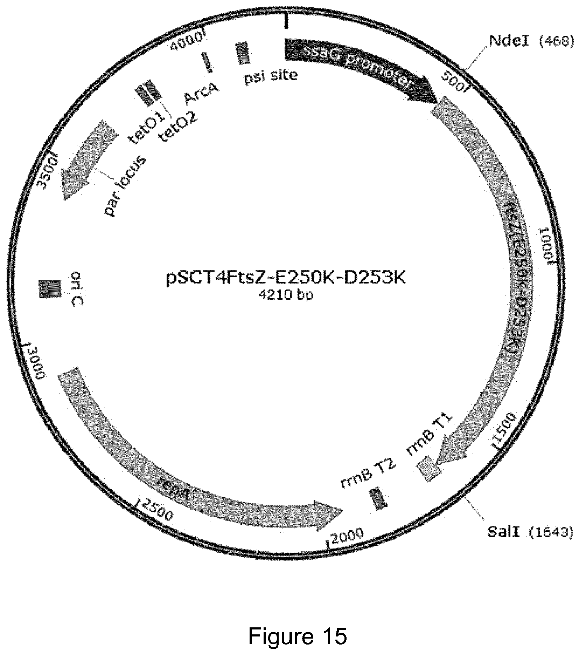

**Figure 15** shows a plasmid map of pSCT4FtsZ-E250K-D253K

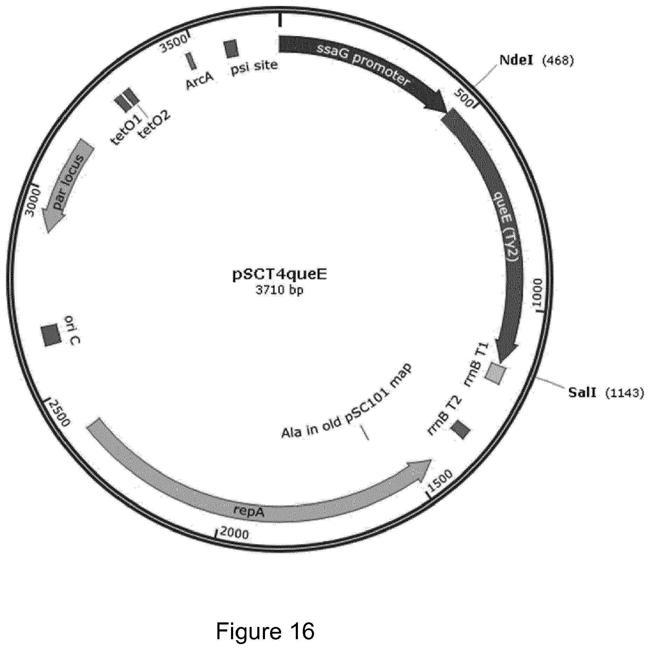

**Figure 16** shows a plasmid map of pSCT4queE

### Detailed description of the invention

The invention is predicated on the surprising discovery that genetically modifying a host bacterium to conditionally repress cell division when the bacterium is internalised into an animal cell attenuates the bacterium without supressing its ability to act as a vaccine delivery vehicle.

As used herein, "conditional repression" refers to the engineering of a genetic modification within the host bacterium to be activated only under certain environmental conditions. In this context, "activated" refers to promoting the phenotype of reduced cell division. The activated modification also causes bacterial filamentation.

Conditional repression is activated when the genetically modified host bacterium (also referred to as the "non-natural bacterium") is internalised into an internal vesicle of an animal cell, such as an endocytic or phagocytic vesicle. The environmental conditions within the internal vesicle activate the one or more genetic modifications that prevent the bacterium from undergoing cell division. This conditional repression has the surprising benefit of attenuating the bacterium, but only within the internal vesicle of the animal cell, without reducing immunogenicity. In fact, the inventors have found that the conditional repression of cell division increases immunogenicity by promoting bacterial filamentation. Without wishing to be bound by theory, filamentation is thought to increase immunogenicity by increasing the surface area upon which heterologous antigen presentation can take place. Thus, the non-natural bacterium of the present invention can be used as an efficacious heterologous antigen vaccine delivery vehicle. Conditional repression has the added benefit of enabling the propagation of the non-natural bacterium under standard laboratory conditions, thus allowing significant quantities of the bacterium to be produced, and furthermore to only attenuate the bacterium when it resides in the most appropriate cellular location for inducing an effective immune response.

Internalisation of bacteria into cells forming part of the body of an animal, especially cells forming the immune system, can increase efficacy of a vaccine when the bacteria are being used to vaccinate an animal against a disease. Likewise, internalisation into cancer cells can form a part of an efficacious immunotherapy against cancer. With regards to cells of the immune system, efficacy of vaccination is particularly increased when the immune cell is an Antigen Presenting Cell (APC), a type of cell which displays on its surface antigen complexed with major histocompatibility complexes. When the bacteria are internalised by an immune cell or by a cancer cell, the cellular location is typically the phagosome.

Therefore as used herein, "animal cell" can refer to any cell, including cancer cells, or fragments of cells, which either forms part of the body of an animal such as a human or other mammal, or derives ancestrally from such a cell. Preferably however, "animal cell" refers to an Antigen Presenting Cell (APC).

Plasmid loss is a well-known problem associated with live bacterial vectors, in which cell division generates plasmid-free daughter cells which do not produce recombinant protein, and therefore have a lighter metabolic burden and come to dominate a culture. It results in a significant reduction in the delivery of the desired non-natural protein. In addition to its main effects, the current invention addresses this problem by reducing cell division and therefore the incidence of plasmid-free daughter cells.

The non-natural bacterium of the invention may be genetically modified or "engineered" to comprise one or more heterologous genes encoding factors that prevent cell division, the expression of which are under the control of an inducible promoter activated when the bacterium is internalised into an animal cell.

As used herein "heterologous gene" refers to a gene that has been introduced into the bacterium, i.e. the introduction of a gene that was not previously present. The gene may be endogenous or exogenous to the bacterium, whereby these terms have their normal meaning in the art. For an endogenous gene, this may comprise introducing an additional copy or copies of said one or more endogenous genes in a heterologous manner. The endogenous gene or genes may also comprise introducing dominant variants of said gene or genes into the host bacterium, whereby "dominant" refers to the ability of the heterologous gene to functionally out-compete the naturally-occurring endogenous counterpart.

Numerous methods and techniques for genetically engineering bacterial strains will be well known to the person skilled in the art. These techniques include those required for introducing heterologous genes into the bacteria either via chromosomal integration or via the introduction of a stable autosomal self-replicating genetic element. Exemplary methods for genetically modifying (also referred to as "transforming" or "engineering") bacterial cells include bacteriophage infection, transduction, conjugation, lipofection or electroporation. A general discussion on these and other methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); which are hereby incorporated by reference.

As used herein "factor" refers to a biological molecule that has an *in vivo* activity. In the context of the present invention, this activity is the ability to influence the process of cell division, for example by promoting or repressing cell division. A factor may be a polypeptide or an RNA, wherein these terms have their conventional meaning in the art. In general, "factor" refers to the biological molecule that possesses the biological activity rather than the gene or DNA sequence encoding the factor itself.

In some embodiments, the one or more heterologous genes encoding factors that prevent cell division are selected from the list consisting of MinC, SulA (Mukherjee et al (1998) PNAS. 95; 2885-90), SlmA (Bernhardt (2005) Mol Cell. 18: 555-64), ZapE (Ortiz et al (2016) FEMS Microbiol Rev. 40: 57-67), QueE (Yadavalli et al (2016) Nature Comm. 7: 12340), DicF (Balasubramanian et al (2016) Mol Biol & Physiol. 1: e00021-15), wild type FtsZ, mutant FtsZ (Stricker (2003) J Bacteriol. 185: 4796-4805; Erickson et al (2001) BMC Microbiology. 1: 7; De Boer et al (2000) J. Bacteriol. 182: 5153-66), ClpXP (Camberg et al (2009) PNAS. 106: 10614-19), mutant YeeUV, or a combination thereof. In preferred embodiments, said one or more heterologous genes comprise MinC.

In some embodiments, MinC comprises the sequence encoded by SEQ ID NO:1 or a functional variant or fragment thereof. In some embodiments, SulA comprises the sequence encoded by SEQ ID NO:3 or a functional variant or fragment thereof. In some embodiments, SimA comprises the sequence encoded by SEQ ID NO:4 or a functional variant or fragment thereof. In some embodiments, ZapE comprises the sequence encoded by SEQ ID NO:5 or a functional variant or fragment thereof. In some embodiments, QueE comprises the sequence encoded by SEQ ID NO:6 or a functional variant or fragment thereof. In some embodiments, DicF comprises the sequence encoded by SEQ ID NO:7 or a functional variant or fragment thereof. In some embodiments, mutant FtsZ comprises the sequence encoded by any one of SEQ ID NOs:8 - 10 or functional variants or fragments thereof. In some embodiments, ClpXP comprises the sequence encoded by SEQ ID NOs:11 or 12 or functional variants or fragments thereof.

Each of these factors prevents cell division and/or induces filamentation of the non-natural bacterium by interfering with the bacterial Min system or a downstream process of septum-production such as formation or attachment of the FtsZ ring. The Min system is required for effective cell division (Rowlett & Margolin (2013) Current Biol. 23: R553-6). For example, MinC, which along with MinD and MinE comprises one of major components of the Min system, is used by the bacterium in combination with FtsZ during cell division to identify the midpoint of the cell. Each component of the Min system operates to generate the dynamic oscillation of FtsZ from the two poles of the bacterium to promote Z-ring formation around the midpoint of the cell. The accurate determination of the midpoint of the cell is essential for enabling cell division into two equally sized progeny. The MinD and MinE components of the Min system bind and localise MinC in a negative gradient from the midpoint to the poles of the cell. As MinC is an inhibitor of FtsZ, the negative gradient allows functional FtsZ to concentrate around the midpoint of the cell to form the Z-ring structure. The introduction and expression of heterologous MinC inhibits the formation of the Z-ring by preventing the localisation of FtsZ to the cellular midpoint, thus repressing cell division.

Other factors, such as SulA and SlmA, also inhibit FtsZ polymerization. Others, such as YeeUV, inhibits FtsZ and MreB polymerization. Particular C-terminal truncation mutants of YeeV prevent binding of YeeV to MreB but not FtsZ (Tan et al (2011) Mol Microbiol. 79: 109-118). These mutants are particularly advantageous as they inhibit cell division but promote the filamentation of the cell. In some embodiments, the YeeV mutant is encoded by the sequence of SEQ ID NO:2.

Other factors, the increased expression of which supresses FtsZ polymerisation include ZapE, DicF and QueE. Other factors, such as ClpXP, degrade essential components of the Min system to prevent cell division. Conditional activation of heterologous ClpXP expression can therefore prevent cell division of the non-natural bacterium.

In some embodiments, one or more of the one or more heterologous genes encode small non-coding RNAs (sRNAs) that repress the expression of one or more endogenous factors that promote cell division. The sequence of each sRNA is complementary to a portion of one mRNA encoding an endogenous factor that promotes cell division. The sRNA may have a varying degree of complementarity to the mRNA. For example, the sRNA may comprise 20 to 30 nucleotides complementary to a region of the mRNA which is to be translationally repressed. In some embodiments, the portion of the sRNA complementary to the mRNA sequence is at least 98% complementary to the target sequence of the mRNA, wherein the % complementarity refers to the exact degree of complementary base-pairing across the region of complementarity. In some embodiments, the region of complementarity in the target mRNA comprises the 5' un-translated region (UTR) of the mRNA. The complementary sequence within the sRNA may be at the 5' end of the sRNA. In general, base-pairing between the sRNA and the 5'UTR of the target mRNA represses translation, for example, by occluding ribosomal binding at the translation initiation site. In some embodiments, the base pairing between the sRNA and the 5' UTR of the mRNA is stabilised by Hfq. Hfq binds to a particular nucleotide sequence in the 3' region of the sRNA to stabilise base-pairing between the sRNA and target mRNA. Thus, in some embodiments, the sRNA will comprise an Hfq binding region 3' to the sRNA sequence complementary to the mRNA. Principles of design and construction of sRNAs are discussed in more detail in: Yoo et al (2013) Nature Protocols. 8: 1694-1707, which is hereby incorporated by reference.

In some embodiments, the one or more heterologous genes are chromosomally integrated or encoded by an autonomous genetic element. Examples of self-replicating autosomal genetic elements (also referred to herein as "vectors" or "replicons") include, but are not limited to, plasmids, to which the one or more heterologous genes may be attached so as to bring about the replication of the heterologous gene or attached segment in the target cell. Suitable genetic elements include those that enable the "stable" replication of the genetic element during cell division, i.e. stable genetic elements are those that allow the genetic element to be maintained in all or predominantly all progeny during successive rounds of cell division.

Suitable genetic elements will vary depending on the identity of the host bacterium from which the non-natural bacterium is derived. Examples of suitable genetic elements include, but are not limited to, plasmids comprising an origin of replication selected from pMB1, ColEl, p15A, pSC101 and RK2, wherein the host bacterium is a gram-negative bacterium, or an origin of replication selected from the group consisting of pWV01 or pUB110, wherein the host bacterium is gram-positive bacteria. Other suitable origins of replication will be well known to the person skilled in the art.

The "genetic elements" described herein comprise a DNA coding sequence operably linked to a promoter. "Operably linked" refers to a juxtaposition wherein the components so described are in a relationship permitting them to function in their intended manner. For instance, a promoter is operably linked to a coding sequence if the promoter affects its transcription or expression.

As used herein, a "promoter" refers to a DNA regulatory region capable of binding RNA polymerase and initiating transcription of a downstream (3' direction) coding or non-coding sequence. A promoter may also be a regulatory DNA sequence that affects the binding of RNA polymerase at the transcription initiation site. For the purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence may be found a transcription initiation site, as well as protein binding domains responsible for the binding of RNA polymerase.

Promoters can be constitutively active (wherein "active" means transcription is "on"), spatially restricted or inducible. As used herein "spatially restricted" refers to a promoter that is only active in a specific subset of cells or cellular compartment of a multicellular organism. A spatially restricted promoter can thus be used to activate the expression of a nucleic acid in a particular tissue or cell type of a multicellular organism.

As used herein an "inducible promoter" refers to a promoter that enables the temporal and/or spatial activation of transcription in response to external physical or environmental stimuli. Inducible promoters include those activated by the presence of specific small molecules that alleviate transcriptional repression. For example, transcription from such an inducible promoter may be regulated by a "repressor protein". As used herein, "repressor protein" refers to a polypeptide that binds to and occupies the inducible promoter to prevent transcription initiation. When bound to the promoter, said repressor protein can prevent binding or recruitment of RNA polymerase or associated co-factors to the transcription initiation site to prevent the activation of transcription. However, upon binding its relevant small molecule, or encountering its relevant physical or environmental stimulus, the repressor protein can no longer bind to the promoter sequence, and thus transcriptional repression is relieved. Examples of repressor proteins include lambda repressor cl and variants thereof (e.g. the thermo-labile mutant cl857 (SEQ ID NO:31), the arabinose repressor AraC (SEQ ID NO:33) and the tetracycline repressor TetR (SEQ ID NO:35) (Bertram &Hillen (2008) Microbial Biotechnol 1:2-16; Rosano & Ceccarelli (2014) Frotniers Microbiol 5: 172; Love et al (1996) Gene 176:49-53).

Other types of inducible promoters are those that activate transcription in response to environmental stimuli. Examples of such inducible promoters include *P_{pagC}, P_{nirB}, P_{ssaG}, P_{sifA}, P_{sifB}, P_{sseA}, P_{sseG}* and *P_{sseJ}* (Dunstan et al. (1999) Infect. Immun. 67: 5133-5141; Xu et al. (2010) Infect. Immun. 78: 4828-4838; Kröger et al. (2013) Cell Host & Microbe 14: 683-695). Suitable inducible promoters for use in the invention are those that activate transcription in response to the environmental conditions of phagocytic vesicles within an animal cell, preferably wherein the animal cell is an antigen presenting cell (APC). For example, such suitable inducible promoters may activate expression of their operably linked genes at a pH in the range of pH 3 to pH 6.5, preferably at a pH in the range of pH 5.0 to pH 6.5, more preferably at a pH of about pH 5.8. The exact inducible promoter most relevant to particular embodiments of the invention will depend on the host bacterium from which the non-natural bacterium is derived. A suitable host-specific inducible promoter can be identified by culturing the host bacterium in so called "InSPl2" conditions, which are fully described in Kröger et al. 2013, Cell Host & Microbe 14: 683-695, incorporated herein by reference. InSPl2 conditions comprise culturing the bacterium in Phosphate Carbon Nitrogen (PCN) medium supplemented with 0.4 mM inorganic phosphate at pH 5.8. PCN medium is a medium designed to induce phagosome-dependent promoters, as described in Löber et al. 2006, Int. J. Med. Microbiol. 296: 435-447, which is hereby incorporated by reference. The bacterium can be cultured until an OD₆₀₀ₙₘ of 0.3 is reached. Deep sequencing and RNA-sequencing of mRNA transcripts can then be combined to ascertain which genes are most preferentially expressed under InSPI2 conditions in the bacterium. When compared with the transcriptome analysis of the bacterium under standard laboratory conditions, such an analysis can be used to reveal inducible promoters most selectively activated under conditions reminiscent of phagocytic vesicles of APCs.

In some embodiments, the one or more heterologous genes may be under the control of an inducible promoter selected from the list consisting of *P_{pagC}, P_{nirB}, P_{ssaG}, P_{sifA}, P_{sifB}, P_{sseA}, P_{sseG}* and *P_{sseJ}* or a combination thereof. Transcription from all of *P_{pagC}, P_{nirB}, P_{ssaG}, P_{sifA}, P_{sifB}, P_{sseA}, P_{sseG}* and *P_{sseJ}* is active in phagosomes. The invention embodies variants and fragments of said inducible promoters that maintain inducible operability within phagosomes, or under InSPl2 conditions. In some embodiments, the functional variants include those having similar or modified sequences to *P_{pagC}, P_{nirB}, P_{ssaG}, P_{sifA}, P_{sifB}, P_{sseA}, P_{sseG}* and *P_{sseJ},* and similar or substantially identical promoter activity as the wild-type promoter from which the variant is derived, particularly with respect to its ability to induce expression *in vivo.* Similar modified sequences may include having at least about 70%, 80%, 85%, 90%, 95%, 96%, 97%, 98% or 99% identity to the wild-type sequence of any of *P_{pagC}, P_{nirB}, P_{ssaG}, P_{sifA}, P_{sifB}, P_{sseA}, P_{sseG}* and *P_{sseJ}.*

In some embodiments, one or more genes encoding endogenous factors that promote cell division are genetically modified in the bacterium to have repressed expression when the bacterium is internalised into an animal cell, i.e. said one or more genes are modified to be conditionally repressed when the bacterium is internalised into an animal cell. Preferably, these one or more endogenous factors will comprise MinD and/or MinE, and repression will be via excision of the MinD and/or MinE gene sequence(s) from the genome, as can be effected by a recombinase acting on suitably placed site-specific recombination sites. Conditional repression can be achieved by engineering said one or more endogenous factors to be selectively removed or transcriptionally repressed when the bacterium is internalised into an animal cell, preferably wherein the non-natural bacterium is internalised into a phagocytic vesicle, preferably wherein the animal cell is an APC.

In some embodiments, the expression of the endogenous factor is conditionally repressed by modifying the 5' and 3' regions flanking the gene sequence of the one or more endogenous factors to comprise site-specific recombination sites (SSRs). When the engineered 5' and 3' SSRs are in the same orientation, in the presence of the cognate recombinase, the intervening sequence between the SSRs will be excised and the two SSRs will recombine, thus resulting in a single SSR in the genome in the absence of the intervening sequence. The principles of site-specific recombination are disclosed in Hallet & Sherratt (1997) FEMS Microbiol. Rev. 21:157-178, which is hereby incorporated by reference. In this embodiment, the non-natural bacterium is further engineered to comprise a heterologous gene encoding a recombinase that recognises and cleaves the engineered SSRs. The expression of the recombinase is under the control of an inducible promoter activated when the bacterium is internalised into an animal cell such as an immune cell or a cancer cell, preferably wherein the non-natural bacterium is internalised into a phagocytic vesicle, and preferably wherein the animal cell is an APC. The inducible promoter may be any of which are described herein. Preferably, the inducible promoter is selected from the list consisting of *P_{pagC}, P_{nirB}, P_{ssaG}, P_{sifA}, P_{sifB}, P_{sseA}, P_{sseG}* and *P_{sseJ}* or functional variants thereof. The heterologous gene may be chromosomally integrated or encoded by an autonomous genetic element.

The 5' and 3' flanking regions of the gene-encoding sequence may be modified to incorporate SSRs using standard genetic engineering techniques. For example, the CRISPR/Cas system may be used to site-specifically cleave the double-stranded DNA at sites immediately adjacent to the region where the SSR is to be inserted. A template strand can be used to guide recombination of the cleaved DNA to insert the desired site-specific recombination sites. The template strand may comprise the SSR flanked by 5' and 3' sequences complimentary to the regions either side of the DNA cleavage site, thus allowing the template-directed insertion of the SSR into the DNA cleavage site.

Desired recombination sites can be added to target genome sites in any way known to the skilled person, for example through the use of targeted homologous recombination protocols employing antibiotic selection steps, such as the lambda Red homologous recombination system.

The site-specific recombination site may be *loxP* (SEQ ID NO:27), wherein the heterologous gene encoding the cognate recombinase comprises Cre (SEQ ID NO:26), or a functional variant thereof. Or, the site-specific recombination site may be *FRT* (SEQ ID NO: 29), wherein the heterologous gene encoding the cognate recombinase comprises Flp (SEQ ID NO:28, or a functional variant thereof.

In some embodiments, the one or more endogenous factors that promote cell division, the expression of which are to be conditionally repressed, are selected from the list consisting of minD, minE, ftsZ, ftsA, zipA, ftsQ, ftsL, ftsl, ftsN. xerC, xerD, ftsK, or a combination thereof, preferably being selected from minD and/or minE.

In some embodiments, the one or more endogenous factors to be excised from the bacterium by conditionally activated site-specific recombination comprises the *dif* site (SEQ ID NO: 22), or a functional fragment thereof. The *dif* site is a 28 bp DNA sequence in the terminus region of the bacterial chromosome that is the target site of site-specific recombinases XerC and XerD. XerCD recombines *dif* to segregate sister chromosomes following chromosome replication. Inhibition of Xer recombination or deletion of *dif* results in filamentation (Kuempel et al (1991), New Biol 3: 799-811).

In some embodiments, the expression of genes encoding said one or more endogenous factors that promote cell division are modified to be under the control of a repressor protein. The endogenous factors may be engineered to be under transcriptional control of the repressor protein by engineering the endogenous transcriptional promoter site to comprise an inducible promoter to which the repressor protein can bind and repress transcription. Said endogenous promoter site can be engineered by using methods conventional in the art such as *de novo* synthesis of the promoter. The non-natural bacterium is further engineered to comprise a heterologous gene encoding the cognate repressor protein. The expression of said repressor protein is under the control of an inducible promoter activated when the bacterium is internalised into an animal cell such as an immune cell or a cancer cell, preferably wherein the cell is internalised into a phagocytic vesicle, preferably wherein the cell is an APC. In some embodiments, the inducible promoter is selected from the list consisting of *P_{pagC}, P_{nirB}, P_{ssaG}, P_{sifA}, P_{sifB}, P_{sseA}, P_{sseG}* and *P_{sseJ}*, or functional variants thereof. In some embodiments, the repressor protein is selected from the list consisting of the lambda repressor cl, the thermo-labile lambda repressor mutant cl857, the arabinose repressor AraC, the tetracycline repressor TetR, or functional variants or fragments thereof. In some embodiments, the repressor protein is encoded one of the sequences selected from the list consisting of SEQ ID NO:31, SEQ ID NO:33 or SEQ ID NO:35. The heterologous gene encoding the repressor protein may be chromosomally integrated or encoded by an autonomous genetic element.

In some embodiments, the non-natural bacterium of the invention is genetically modified such that one or more genes encoding endogenous factors that repress cell division are under the control of an inducible promoter activated when the bacterium is internalised into an animal cell, preferably wherein the bacterium is internalised into a phagocytic vesicle, preferably wherein the animal cell is an APC.

In some embodiments, the one or more endogenous factors that repress cell division are selected from the list consisting of MinC, SulA, SlmA, ZapE, QueE, DicF, ClpXP, or a combination thereof, but preferably comprise MinC. The inducible promoter may be selected from the list consisting of *P_{pagC}, P_{nirB}, P_{ssaG}, P_{sifA}, P_{sifB}, P_{sseA}, P_{sseG}* and *P_{sseJ}*, or functional variants thereof.

In some embodiments, the inducible promoter used in any of the forgoing embodiments of the invention comprises SEQ ID NO: 36 or an operable variant thereof.

In some embodiments the bacterium is further modified to partially or completely suppress expression of one or more chromosomally-encoded genes, with the effect of attenuating the bacterium. In these embodiments, the activity of the suppressed gene(s) is instead provided by a complementary copy which is encoded on the same autonomous genetic element encoding the one or more other heterologous genes of the invention. Preferably, these other heterologous genes are those encoding the one or more factors that prevent cell division. Preferably, the complementary copy is under the control of its native promoter, i.e. the promoter which activates the transcription of the endogenous essential gene *in vivo.* Such a strategy ensures that if a bacterium loses the autonomous genetic element suppressing cell division, thereby recommencing cell division, it will be attenuated.

In some embodiments the one or more essential genes is selected from the list consisting of *aroA, aroC, purA, purE, asd, cya, crp, clpX, galE, guaA, guaB, htrA, msbB, phoP, phoQ, pmi, pur* or a combination thereof. The attenuation of the essential gene can be achieved by excising the chromosomal gene, or introducing attenuating mutations into the coding sequence of the essential gene, or by repressing the expression of the essential gene. In some embodiments, the host bacterium will comprise basal attenuating mutations or deletions of the one or more essential genes. In some embodiments, wherein the host bacterium is derived from *Salmonella spp.* the one or more essential genes may be additionally selected from *ssaV, ssaJ, ssaK, ssaU, ssaL, ssaM, ssaO, ssaP, ssaQ, ssaR, ssaS, ssaT, ssaD, ssaE, ssaG, ssal, ssaC,* and *ssaH.* The essentiality of each of these genes is described in WO 2009/158240, which is hereby incorporated by reference.

In some embodiments, the non-natural bacterium of the invention is further engineered to express a heterologous gene encoding a heterologous antigen. As used herein, an "antigen" refers to a molecule that is capable of inducing an immune response in a host. The heterologous antigen may be under to control of a constitutive, spatially-restricted, or inducible promoter. The inventors have surprisingly found that the conditional repression of cell division coupled with increased filamentation of the non-natural bacterium attenuates the bacterium and improves immunogenicity. Thus, the non-natural bacterium of the invention is an effective vaccine carrier.

In preferred embodiments the non-natural bacterium expresses an antigenic protein encoded by, for example, a plasmid it carries which encodes a prokaryotic promoter operably linked with a heterologous gene. In some embodiments, however, the heterologous gene may represent the delivery system for a DNA vaccine. In these embodiments, the heterologous gene is encoded on a genetic element such as a plasmid, and is operably linked with a promoter which functions in animal cells. Upon uptake of the genetic element by an animal cell such as an immune cell or cancer cell, the animal cell's native expression machinery internally produces the antigenic protein, and the effects of vaccination are thereby elicited.

The ability of the non-natural bacterium to induce an immune response against the expressed heterologous antigen can be tested by, for example, immunising animal hosts such as mice. The bacterial vaccine can be administered via, for example, the orogastric, intranasal or intraperitoneal routes. The bacteria then invade or are phagocytosed by antigen-presenting cells which induce antigen expression, leading to the generation of an immune response against said antigen by presentation via Major Histocompatibility Complex (MHC) Class I and II pathways. Numerous tests of this process and the concomitant immunity are known to the skilled person.

In some embodiments, the heterologous antigen is derived from a pathogenic organism selected from the group consisting of virus, bacterium, fungus, or other eukaryotic parasite.

Wherein the heterologous antigen is derived from a virus, the heterologous antigen may be derived from a virus selected from the list consisting of Dengue Fever Virus, Ebola Virus, Encephalomyocarditis Virus, Hepatitis A, B and C Viruses, Herpes Simplex Virus, Human Enterovirus, Human Immunodeficiency Virus, Human Papilloma Virus, Human Parvovirus, Human Rhinovirus, Influenza Virus, Lymphocytic Choriomeningitis Virus, Middle East Respiratory Syndrome Virus, Mouse Mammary Tumour Virus, Rabies Virus, Respiratory Syncytial Virus, Severe Acute Respiratory Syndrome Coronavirus, West Nile Virus, Zika Virus or a combination thereof. Conventional heterologous antigens derived each of these viruses capable of inducing immune responses in a host are well known to the skilled person.

Wherein the heterologous antigen is derived from a bacterium, the heterologous antigen may be derived from a bacterium selected from the list consisting of *Aerococcus* spp., *Aeromonas hydrophila, Actinobaculum schaalii, Acinetobacter baumannii, Aggregatibacter actinomycetemcomitans, Alloscardovia omnicolens, Anaplasma phagocytophilum, Bacillus* spp. (including *B. anthracis* and *B. cereus), Bacteroides fragilis, Bartonella* spp. (including *B. henselae*), *Bordetella* spp. (including *B. pertussis*), *Borrelia* spp. (including *B. burgdorferi*)*, Brucella* spp. (including *B. abortus*), *Burkholderia* spp. (including *B. cepacia*), *Campylobacter jejuni, Chlamydia* spp. (including *C. trachomatis*), *Chlamydophila pneumoniae, Clostridium* spp. (including *C. botulinum, C. difficile* and *C. tetani*)*, Corynebacterium diptheriae, Enterobacter* spp. (including *E. aerogenes* and *E. cloacae*), *Enterococcus* spp. (including *E. faecalis* and *E. faecium*), *Escherichia coli* (including ETEC and O157:H7), *Francisella tularensis, Haemophilus influenzae, Helicobacter pylori, Klebsiella pneumoniae, Legionella pneumophila, Leptospira* spp., *Listeria monocytogenes, Moraxella catarrhalis, Mycobacterium* spp. (including *M. avium, M. bovis* and *M. tuberculosis*), *Mycoplasma pneumoniae, Neisseria* spp. (including *N. gonorrhoeae* and *N. meningitidis*), *Neoehrlichia mikurensis, Nocardia* spp., *Proteus mirabilis, Pseudomonas* spp. (including *P. aeruginosa* and *P. stutzeri*)*, Rickettsia* spp., *Salmonella* spp. (including *S. enterica*), *Serratia marcescens, Shigella* spp. (inclusing *S. dysenteriae, S. flexneri* and *S. sonnei*)*, Staphylococcus* spp. (including *S. aureus* and *S. epidermis*), *Stenotrophomonas maltophilia, Streptococcus* spp. (including *S. pyogenes*), *Treponema pallidum, Tropheryma whipplei, Ureaplasma urealyticum, Vibrio cholera, Yersinia* spp. (including *Y. pestis* and *Y. pseudotuberculosis*). or combinations thereof. Conventional heterologous antigens derived each of these bacteria capable of inducing immune responses in a host are well known to the skilled person.

Wherein the heterologous antigen is derived from a fungus, the heterologous antigen may be derived from a fungus selected from the list consisting of *Aspergillus* spp. (including *A. niger*), *Blastomyces* spp., *Candida* spp. (including *C. albicans, C. auris* and *C. tropicalis*), *Coccidioides immitis, Cryptococcus neoformans, Gibberella moniliformis, Histoplasma capsulatum, Fonsecae pedrosoi, Fusarium oxysporum, Microsporum* spp., *Pneumocystis jirovecii, Rhizopus oryzae* and *Trichophyton* spp. Other eukaryotic pathogens include *Acanthamoeba* spp., *Ascaris lumbricoides, Babesia* spp., *Bruga* spp. (including *B. malayi* and *B. timori*)*, Cryptosporidium parvum, Entamoeba histolytica, Giardia lamblia, Leishmania* spp. including (*L. donovani* and *L. major*), *Necator americanus, Parachlamydia acanthamoebae, Plasmodium* spp. (including *P. falciparum* and *P. vivax*) *Toxoplasma gondii, Trichinella spiralis, Trichomonas vaginalis, Trypanosoma* spp. (including *T. cruzi* and *T. brucei*) and *Wuchereria bancrofti.* Conventional heterologous antigens derived each of these fungi capable of inducing immune responses in a host are well known to the skilled person.

In some embodiments, the heterologous antigen is a cancer vaccine. Exemplary cancer antigens include tumour-specific proteins, tumour-associated proteins, growth factors or angiogenesis factors. In some embodiments, the cancer antigen may be selected from the list consisting of AFP (alpha-fetoprotein), CA-125 (ovarian cancer antigen 125), CEA (carcinoembryonic antigen), endoglin (CD105; including transforming growth factor-beta receptor: TGF-beta), ETA (epithelial tumour antigen), Her2/neu (receptor tyrosine-protein kinase erbB-2), HMW-MAA (human high molecular weight-melanoma-associated antigen), ISG15 (interferon-stimulated gene 15), MAGE (melanoma-associated antigen), MUC1 (mucin 1, cell surface associated), NY-ESO-1 (New York esophageal squamous cell carcinoma 1), p53, p60, PSA (prostate-specific antigen), survivin (an apoptosis protein), TRP2 (tyrosinase-related protein 2), VEGFR2 (vascular endothelial growth factor receptor 2), or antigenic fragments thereof. As defined herein, "antigenic fragment" refers to a portion of the antigen that is capable of inducing an immune response in the host.

In some embodiments, the host bacterium from which the non-natural bacterium is derived is selected from the list of genera consisting of *Bacillus* spp. (including *B. subtilis*) *Bifidobacterium* spp., *Clostridium* spp., *Lactobacillus* spp., *Lactococcus* spp., *Listeria* spp. (including *L. monocytogenes*) or *Mycobacterium* spp. (including *M. bovis, M. smegmatis* or *M. tuberculosis*), *Bordetella* spp. (including *B. pertussis*), *Pseudomonas* spp. and members of the family Enterobacteriaceae: *Citrobacter* spp., *Escherichia* spp. (including *E. coli*), *Klebsiella* spp., *Proteus* spp., *Salmonella* spp., *Serratia* spp. *Shigella* spp. (including *S. dysenteriae, S. flexneri* and *S. soneii*)*, Vibrio* spp (including *V. cholerae*) and *Yersinia* spp. In some embodiments, the bacterium is selected from the genus *Salmonella,* preferably wherein the bacterium is *Salmonella enterica* serovar Typhimurium, Typhi or Paratyphi. In some embodiments, the host bacterium may be derived from a *Salmonella enterica* with a basal mutation in *aroA, aroC,* ssaV, or a combination thereof, whereby "basal" refers to the bacterium already comprising the attenuating mutation within the endogenous gene.

In a second aspect of the invention, there is provided the non-natural bacterium for use in the treatment or prevention of a disease. In some embodiments, the non-natural bacterium is for use in the treatment or prevention of a disease in an animal. In some embodiments, the animal is a human.

In some embodiments, the disease to be treated or prevented is cancer. The cancer may be selected from the list consisting of Adenoid Cystic Carcinoma, Adrenal Gland Tumour, Amyloidosis, Anal Cancer, Ataxia-Telangiectasia, Atypical Mole Syndrome, Beckwith Wiedemann Syndrome, Bile Duct Cancer, Birt Hogg Dube Syndrome, Bladder Cancer, Bone Cancer, Brain Tumour, Breast Cancer, Carcinoid Tumour, Carney Complex, Cervical Cancer, Colorectal Cancer, Ductal Carcinoma, Endometrial Cancer, Oesophageal Cancer, Familial-Adenomatous Polyposis, Gastric Cancer, Gastrointestinal Stromal Tumour, HER2-Positive Breast Cancer, Hereditary Prostate Cancer, Islet Cell Tumour, Juvenile Polyposis Syndrome, Kaposi's Sarcoma, Kidney Cancer, Laryngeal Cancer, Acute Lymphoblastic Leukaemia, Acute Myeloid Leukaemia, Chronic Lymphocytic Leukaemia, Chronic Myeloid Leukaemia, Acute Lymphocytic Leukaemia, Liver Cancer, Lobular Carcinoma, Lung Cancer, Small Cell Lung Cancer, Hodgkin's Lymphoma, Non-Hodgkin's Lymphoma, Lynch Syndrome, Malignant Glioma, Mastocytosis, Melanoma, Meningioma, Multiple Endocrine Neoplasia Type 1, Multiple Endocrine Neoplasia Type 2, Multiple Myeloma, Myelodysplastic Syndrome (MDS), Nasopharyngeal Cancer, Neuroendocrine Tumour, Nevoid Basal Cell Carcinoma Syndrome, Oral Cancer, Osteosarcoma, Ovarian Cancer, Pancreatic Cancer, Pancreatic Neuroendocrine Tumours, Parathyroid Cancer, Penile Cancer, Peritoneal Cancer, Peutz-Jeghers Syndrome, Pituitary Gland Tumour, Pleuropulmonary Blastoma (Childhood), Polycythemia Vera, Prostate Cancer, Renal Cell Cancer, Retinoblastoma (Childhood), Salivary Gland Cancer, Sarcoma, Sarcoma - Alveolar Soft Part and Cardiac, Sarcoma - Kaposi, Skin Cancer (Non-Melanoma), Small Bowel Cancer, Small Intestine Cancer, Small Intestine Cancer, Stomach Cancer, Testicular Cancer, Thymoma, Thyroid Cancer, Turcot Syndrome, Uterine (Endometrial) Cancer, Vaginal Cancer, Von-Hippel-Lindau Syndrome and Wilms' Tumour (Childhood).

In some embodiments, the heterologous gene encoding the tumour antigen is cognate to the cancer to be treated. In other words, the heterologous tumour antigen can be a tumour-specific antigen expressed in the tumour cells of the cancer to be treated. In some embodiments, the heterologous tumour antigen is a tumour-associated antigen (TAA), whereby a "TAA" refers to an antigen that is expressed at elevated levels in the tumour cells.

In another aspect of the invention, there is provided the use of the non-natural bacterium of the first aspect of the invention in the vaccination of a subject against a disease. The disease may be any of those as described in the second aspect of the invention.

In another aspect of the invention, there is provided a method of manufacturing the non-natural bacterium according to any of the previous aspects of the invention, by introducing the genetic modifications disclosed herein.

The invention is illustrated by the following non-limiting examples:

### Example 1

To construct the low copy number expression plasmid pSCT4minCOX, primers minCOXF and minCOXR were used to amplify the *minC* gene of SL3261 genomic DNA. The PCR product and expression plasmid pSCT4c were digested using Ndel and Sail and ligated to generate the precursor plasmid pSCT4cminCOX. pSCT4cminCOX has *minC* under the control of the phagosome-induced SPI-2 promoter *P_{ssaG}*, which is not active in nutrient broths such as LB or TB. PCN (Phosphate Carbon Nitrogen minimal medium) with a low concentration of inorganic phosphate was used to induce the *Salmonella* SPI-2 regulon. An *E. coli pepA* mutant strain was used for cloning operations. The pSCT4cminCOX plasmid was transformed into S. Typhimurium SL3261, and transformants were selected on LB agar plates supplemented with 20 µg/ml chloramphenicol. Single colonies were isolated and cultured overnight in LB broth in the absence of antibiotic. Xer recombination resulted in the deletion of the *cat* gene to generate chloramphenicol-sensitive colonies of SL3261 (pSCT4minCOX).

To evaluate the effect of *minC* overexpression in the morphology of bacteria, a single colony of SL3261 (pSCT4minCOX) was inoculated into LB broth and incubated for 16 hours at 37°C and 200 rpm. This starter culture was used to inoculate PCN medium (pH 5.8) which was incubated for 6 hours at 37°C and 200 rpm. A volume of culture equivalent to an optical density of A₆₀₀ = 2 was collected and washed in PBS. A small volume of cells were applied on a microscope slide and observed under the microscope (Figure 6).

The results show the repressed cell division and induced filamentation of the non-natural bacterium comprising the MinC expression plasmid after 6 h. The control bacterium does not show this phenotype.

### Example 2

The RAW 264.7 murine macrophage cell line was cultured in DMEM containing 2 mM L-glutamine and 10% Foetal Bovine Serum. Cells were seeded at a density of 2 x 10⁵ cells per well in 24-well plates containing glass coverslips and grown for approximately 30 hours. RAW 264.7 cells were infected with S. Typhimurium SL3261 (pSCT4minCOX), incubated for 2 or 16 hours, washed with PBS and fixed using 3% formalin. RAW 264.7 cells were permeabilised using 0.1% Triton X-100 and subsequently blocked using Purified Rat Anti-Mouse CD16/CD32 (Mouse BD Fc Block) containing 3% Bovine Serum Albumin for 1 hour. Bacterial cells were then stained using an anti-LPS monoclonal antibody (IE6) directly conjugated to Dylight 488 fluorochrome for 30 minutes (Figure 7).

RAW 264.7 cells were also infected with S. Typhimurium strains MD58 (*aroC*) and WT05 (*aroC, ssaV*) containing plasmid pBRT1RFP in which Red Fluorescent Protein (RFP, SEQ ID NO: 40) is under the control of the *P_{ssaG}* promoter. The gene encoding monomeric red fluorescent protein was codon-optimised for expression in *E. coli* and synthesised in plasmid pMA-mRFP. The 695bp fragment containing the RFP cistron was cloned into plasmid pBRT1 Nc as an Ndel-Sall fragment. S. Typhimurium strains for infection were used in parallel that additionally contained pSCT4minCOX (Figure 8).

Coverslips were mounted on glass microscope slides using Poly-Mount (Polysciences) and visualised using a fluorescent microscope (Zeiss Axiophot) with attached Zeiss Axiocam camera. Images were captured at 400x magnification unless otherwise stated. Fluorescence imaging shows that at the 16 h time point the non-natural bacterium show the induced filamentation phenotype within the RAW 264.7 cells.

### Example 3

Low copy number expression plasmids containing sequences encoding *queE* and three selected mutant variants of *ftsZ* under the control of phagosome-induced promoter *P_{ssaG}* were constructed as follows. A 960 bp DNA fragment containing the sequence encoding 314 N-terminal amino acids of FtsZ was amplified by PCR from *Salmonella* Typhi ZH9 genomic DNA using primers ftsZ(1-314)Ndel and ftsZ(1-314)Xbal. The PCR product was digested with Ndel-Xbal and cloned into pSCT4c to generate plasmid pSCT4cFtsZ-1/314. Plasmids pSCT4cFtsZ-D45A and pSCT4cFtsZ-E250K-D253K contain the DNA sequences encoding for respectively ftsZ mutant cistrons FtsZ(D45A) and FtsZ(E250K-D253K). The DNA fragments encoding two selected ftsZ cistrons were synthesised and cloned in plasmids pUC57-kan-ftsZ-D45A and pUC57-kan-ftsZ-E250K-D253K. The 1175bp Ndel-Sall inserts were subcloned into pSCT4c to generate final plasmids pSCT4cFtsZ-D45A and pSCT4cFtsZ-E250K-D253K. To construct plasmid pSCT4cqueE, primers queE-Ndel and queE-Sall were used to amplify queE gene from ZH9 genomic DNA. The 688bp PCR product was digested with Ndel-Sall and cloned into pSCT4c to generate final plasmid pSCT4cqueE. An *E. coli pepA* mutant strain was used as host in all cloning operations. *Salmonella* Typhimurium strain MD58 was transformed with the recombinant expression plasmids and transformants were selected on LB-aro mix plates supplemented with 20 µg/ml chloramphenicol. Single colonies were isolated and cultured overnight in LB-aro mix broth in the absence of antibiotic selection to allow deletion of the chloramphenicol acetyltransferase (*cat*) gene via Xer recombination and generation of the following final strains: MD58(pSCT4FtsZ-1/314), MD58(pSCT4FtsZ-D45A), MD58(pSCT4FtsZ-E250K-D253K) and MD58(pSCT4queE).

To study the effect on cell division of *ftsZ* mutants and of *queE* overexpression, single colonies of the four MD58 derived strains above described were inoculated in LB-aro and incubated for 20 hours at 37°C. MD58 strain with no plasmid was included as a negative control. The overnight LB-aro mix cultures were used to inoculate PCN medium (pH 5.8) for *P_{ssaG}* induction. After 7 hours growth at 37°C in PCN medium, 10 µl of bacterial cultures were applied to a microscope slide and visualised in phase contrast using a Zeiss Axiophot microscope at 1000x magnification (Figure 9). Control strain MD58 grew normally in PCN and maintained a rod-shaped morphology. However, expression of all three FtsZ mutants and of QueE resulted in a filamentous cellular morphology.

## Claims

1. A genetically engineered non-natural bacterium comprising one or more of the following genetic modifications:
(i). one or more heterologous genes encoding factors that prevent cell division, the expression of which are under the control of an inducible promoter activated when the bacterium is internalised into the intracellular vesicle of an animal cell;
(ii). one or more endogenous factors that promote cell division, the expression of which are genetically modified to be repressed and/or prevented when the bacterium is internalised into the intracellular vesicle of an animal cell; and/or
(iii). one or more endogenous factors that repress cell division, the expression of which are genetically modified to be under the control of an inducible promoter activated when the bacterium is internalised into the intracellular vesicle of an animal cell.

2. The bacterium of claim 1, wherein the 5' and 3' regions flanking the gene sequence of the one or more endogenous factors that promote cell division are modified to comprise site-specific recombination sites, and wherein the bacterium is further engineered to comprise a heterologous gene encoding a recombinase that recognises and cleaves the site-specific recombination sites, wherein the expression of said recombinase is under the control of an inducible promoter activated when the bacterium is internalised into the intracellular vesicle of an animal cell, preferably wherein the said one or more endogenous factors that promote cell division comprise or are operably linked to MinD and/or MinE.

3. The bacterium of any preceding claim, wherein the expression of said one or more endogenous factors that promote cell division are modified to be under the control of a repressor protein, and wherein the bacterium is further engineered to comprise a heterologous gene encoding said repressor protein, the expression of which being under the control of an inducible promoter activated when the bacterium is internalised into the intracellular vesicle of an animal cell.

4. The bacterium of any preceding claim, wherein the bacterium is to be internalised into the intracellular vesicle of an antigen-presenting cell or a cancer cell.

5. The bacterium of any previous claim, wherein the said one or more heterologous genes encoding factors that prevent cell division encode factors selected from the group consisting of MinC, SulA, SlmA, ZapE, QueE, DicF, mutant FtsZ, wild type FtsZ, ClpXP, mutant YeeUV, or a combination thereof, preferably wherein the factor is MinC.

6. The bacterium of any preceding claim, wherein said one or more heterologous genes encode small RNAs that repress the expression of one or more endogenous factors that promote cell division.

7. The bacterium of any preceding claim, wherein the one or more endogenous factors that promote cell division are selected from the group consisting of minDE, ftsZ, ftsA, zipA, ftsQ, ftsL, ftsl, ftsN, xerC, xerD, ftsK, or a combination thereof, preferably wherein the factor is minDE, and the expression of minDE is to be repressed and/or prevented by excision of its gene sequence.

8. The bacterium of any preceding claim, wherein the inducible promoter is selected from the list consisting of *P_{pagC}, P_{nirB}, P_{ssaG}, P_{sitA}, P_{sifB}, P_{sseA}, P_{sseG}, P_{sseJ}* or a combination thereof.

9. The bacterium of any preceding claim, wherein the inducible promoter comprises SEQ ID NO: 36 or an operable variant thereof.

10. The bacterium of any preceding claim, further engineered to carry, and preferably express, a heterologous gene encoding a heterologous antigen.

11. The bacterium of any preceding claim, wherein the one or more heterologous genes are chromosomally integrated or encoded by an autonomous genetic element.

12. The bacterium of any preceding claim, wherein the bacterium is further modified to partially or completely suppress expression of one or more chromosomally-encoded genes, the lack of which would attenuate the bacterium, wherein the activity of the one or more chromosomally-encoded genes is replaced by a functioning complement of the one or more chromosomally-encoded genes encoded by an autonomous genetic element, preferably wherein the autonomous genetic element is that comprising the one or more heterologous genes of any preceding claim, preferably wherein the heterologous genes encode factors that prevent cell division, preferably
wherein the one or more essential genes is selected from the list consisting of *aroA, aroC, ssaV, purA, purE, asd, cya, crp, clpX, galE, guaA, guaB, htrA, msbB, phoP, phoQ, pmi, pur,* or a combination thereof.

13. The bacterium of any preceding claim, wherein the bacterium from which the non-natural bacterium is derived is *Salmonella enterica.*

14. The non-natural bacterium of any preceding claim for use in the treatment or prevention of a disease, including cancer.

15. The use of the non-natural bacterium of any preceding claim in the vaccination of a subject against a disease.

16. A method of manufacturing a non-natural bacterium according to any of claims 1 to 14, comprising introducing into a bacterium any of the genetic modifications of claims 1 to 12.
